(19) Europäisches Patentamt
European Patent Office
Office européen des brevets

(11) Publication number: **0 302 792 B1**

(12) # EUROPEAN PATENT SPECIFICATION

(45) Date of publication of patent specification: **26.10.94**

(51) Int. Cl.[5]: **C07D 213/71**, C07D 213/70, C07D 215/36, C07C 317/22, C07D 231/18, C07D 471/04, C07D 209/30, A61K 31/435, A61K 31/44, C07D 235/22, C07D 307/64

(21) Application number: **88402031.4**

(22) Date of filing: **03.08.88**

The file contains technical information submitted after the application was filed and not included in this specification

(54) **Alkylaminoalkoxyphenyl derivatives, process of preparation and compositions containing the same.**

(30) Priority: **07.08.87 US 82554**

(43) Date of publication of application:
**08.02.89 Bulletin 89/06**

(45) Publication of the grant of the patent:
**26.10.94 Bulletin 94/43**

(84) Designated Contracting States:
**AT BE CH DE ES FR GB GR IT LI LU NL SE**

(56) References cited:
**FR-A- 2 341 578**
**FR-A- 2 400 515**
**US-A- 3 330 831**
**US-A- 4 117 128**

(73) Proprietor: **SANOFI**
**32-34, rue Marbeuf**
**F-75008 Paris (FR)**
(84) Designated Contracting States:
**CH DE ES FR GB GR IT LI LU NL SE AT**

(73) Proprietor: **S.A. SANOFI - PHARMA N.V.**
**Avenue De Béjar, 1**
**B-1120 Bruxelles (BE)**
(84) Designated Contracting States:
**BE**

(72) Inventor: **Gubin, Jean**
**Avenue du Mutsaard 70/B.36**
**B-1020 Brussels (BE)**
Inventor: **Chatelain, Pierre**
**Avenue du Haras 111**
**B-1150 Brussels (BE)**
Inventor: **Inion, Henri**
**Molenweg 101**
**B-1810 Wemmel (BE)**

EP 0 302 792 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

JOURNAL OF MEDICINAL CHEMISTRY, vol. 15, no. 5, 1972, pages 523-529, Columbus,Ohio, US; C.J. SHARPE et al.: "Basic ethers of 2-anilinobenzothiazoles and 2-anilinobenzoxazoles as potential antidepressants"

JOURNAL OF MEDICINAL CHEMISTRY, vol. 21, no. 2, 1978, pages 182-188, Columbus,Ohio, US; M.T. COX et al.: "Linked aryl aryloxypropanolamines as a new class oflipid catabolic agents"

Inventor: **Lucchetti, Jean**
**Rue des Combattants 23A**
**B-5860 Chastre (BE)**
Inventor: **Mahaux, Jean-Marie**
**Allée de Provence 4**
**B-1140 Brussels (BE)**
Inventor: **Vallat, Jean-Noel**
**Rue Alfred Dumeril 72**
**F-31400 Toulouse (FR)**

(74) Representative: **Le Guen, Gérard et al**
**CABINET LAVOIX**
**2, place d'Estienne d'Orves**
**F-75441 Paris Cédex 09 (FR)**

## Description

The present invention relates to new carbocyclic or heterocyclic derivatives and to a process for preparing them.

More particularly, the invention relates to the novel aminoalkoxyphenyl derivatives represented by the general formula :

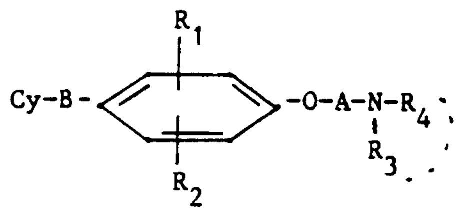

(1)

in which :

B represents a -S-, -SO- or $-SO_2-$ group,

$R_1$ and $R_2$, which are identical or different, each denote hydrogen, a methyl or ethyl radical or a halogen such as chlorine, bromine or iodine,

A denotes a straight- or branched-alkylene radical having from 2 to 5 carbon atoms or a 2-hydroxypropylene radical in which the hydroxy is optionally substituted by a lower alkyl radical,

$R_3$ denotes hydrogen or an alkyl radical,

$R_4$ denotes hydrogen or an alkyl radical or $R_3$ and $R_4$ when taken together, denote an alkylene or alkenylene radical having from 3 to 6 carbon atoms and optionally substituted with a phenyl radical or optionally interrupted by -O-, N= or

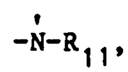

$R_{11}$ denoting hydrogen or a lower alkyl, phenyl, diphenylmethyl, benzyl or halogenobenzyl radical,

Cy is selected from the group consisting of phenyl optionally substituted with R, in the α position with respect to the methine group directly linked to B;
furyl and isoxazolyl optionally substituted with R in the α position with respect to the methine group directly linked to B and further optionally substituted with one or more groups selected from $C_1$-$C_4$ alkyl and phenyl groups;
benzimidazol-1-yl optionally substituted with R in the second position;
benzimidazol-2-yl optionally substituted with R in the α position with respect to the methine group directly linked to B and further optionally substituted on a nitrogen atom by a radical selected from $(C_1$-$C_4)$alkyl, phenyl, diphenylmethyl, benzyl or halogenobenzyl; and
indolizin-2-yl, indolizin-3-yl, pyrazolo[1,5-a]pyridyl, indolyl and quinolyl optionally substituted with R in the α position with respect to the methine group directly linked to B.

R represents hydrogen, an alkyl radical, or a cycloalkyl radical.

In the present context, both in the description and in the claims, the following meaning attaches to the terms stated above:

"alkyl" denotes straight- or branched-saturated aliphatic hydrocarbon residues having up to 8 carbon atoms, such as methyl, ethyl, n-propyl, isopropyl, n-butyl, isobutyl, tert-butyl, n-pentyl, neopentyl, n-hexyl, n-heptyl or n-octyl,

"lower alkyl" denotes saturated aliphatic hydrocarbon residues having up to 4 carbon atoms, such as methyl, ethyl, n-propyl, isopropyl, n-butyl, iso-butyl, tert-butyl or 1-methylpropyl,

"lower alkoxy" denotes a hydroxy group substituted with a lower alkyl group as defined above,

"cycloalkyl" denotes an alicyclic ring having from 3 to 6 carbon atoms.

Thus, taking into account the meanings given above:

R can denote, in particular, a methyl, ethyl, n-propyl, isopropyl, n-butyl, isobutyl, tert-butyl,

1-methylpropyl, n-pentyl, neopentyl, phenyl, monofluoro-, monochloro- or monobromophenyl, difluoro-, dichloro- or dibromophenyl, monomethyl- or dimethylphenyl, or monomethoxy- or dimethoxyphenyl radical , a methylphenyl radical substituted with a halogen atom or a cyclopropyl radical,

A can denote, in particular, a 1,2-ethylene, 1,3-propylene, 2-methyl-1,3-propylene, 1,4-tetramethylene or 1,5-pentamethylene chain,

$R_3$ can denote, in particular, a methyl, ethyl, n-propyl, isopropyl, n-butyl, isobutyl, tert-butyl, 1-methyl-propyl, n-pentyl, n-hexyl, n-heptyl or n-octyl radical,

$R_4$ can denote, in particular, a methyl, ethyl, n-propyl, isopropyl, n-butyl, isobutyl, tert-butyl, n-pentyl, neopentyl, n-hexyl, n-heptyl or n-octyl radical,

$R_3$ and $R_4$, taken together, can denote, in particular, a 1,4-tetramethylene, 1,5-pentamethylene, 3-oxo-1,5-pentamethylene, 3-aza-1,5-pentamethylene,3-methyl-aza-1,5-pentamethylene, 3-phenylaza-1,5-pentamethylene or -CH=CH-N=CH- radical, so that $R_3$ and $R_4$, taken with the nitrogen atom to which they are attached, can denote, in particular, pyrrolidinyl, piperidyl, morpholinyl, piperazinyl, 4-methylpiperazinyl, 4-phenylpiperazinyl or 1H-imidazolyl radical.

A particularly valuable class of compounds of formula (1) are those in which Cy represents an indolizin-3-yl group.

Another class of compounds are those in which $R_1$ and $R_2$ each are hydrogen.

Particularly useful compounds of formula (1) are those in which $R_3$ represent hydrogen and $R_4$ represents tert-butyl or $R_3$ and $R_4$ each represent n-propyl or n-butyl.

Other valuable compounds of formula (1) are those in which R represents an isopropyl or cyclopropyl group.

The invention also relates to the pharmaceutically acceptable salts of the compounds of formula (1) formed with an organic or inorganic acid.

As examples of organic salts of this type, there may be mentioned the oxalate, maleate, fumarate, methanesulfonate, benzoate, ascorbate, pamoate, succinate, hexamate, bismethylenesalicylate, ethanedisulphonate, acetate, propionate,tartrate, salicylate, citrate, gluconate, lactate, malate, cinnamate, mandelate, citraconate, aspartate, palmitate, stearate, itaconate, glycolate, p-aminobenzoate, glutamate, benzenesulphonate and theophyllineacetate, as well as the salts formed with an amino acid such as the lysine or histidine salt.

As examples of inorganic salts of this type, the hydrochloride, hypobromide, sulphate, sulphamate, phosphate and nitrate may be mentioned.

Another object of the invention relates to the N-oxide derivatives of the compounds of formula (1).

The compounds of formula (1) can exist, in some cases, in the form of optical isomers, in particular as a result of the asymetric carbon present when A represents a 2-hydroxypropylene chain.

The invention relates, at the same time, to all the isomers of the compounds of formula (1), the isomers being considered in the dextrorotatory or laevorotatory form, or in the form of a mixture, for example in the form of a racemic mixture.

It has been found that the aminoalkoxyphenyl derivatives of the invention possess exceptional pharmacological properties, especially calcium transport inhibitory properties, as well as bradycardic, hypotensive and antiadrenergic properties.

From this viewpoint, the preferred compounds of the invention are those in which B represents a $-SO_2-$ group.

These properties are capable of making the compounds in question very useful on the treatment of certain pathological syndromes of the cardio-vascular system, especially in the treatment of angina pectoris, hyper-tension,arrhythmia and cerebral circulatory insufficiency.

In the antitumour field, the compounds of the invention may be useful as potentiators of anticancer drugs.

Consequently, the invention also relates to pharmaceutical or veterinary compositions containing, as active principle, at least one aminoalkoxyphenyl derivative of formula (1) or a pharmaceutically acceptable salt of this derivative, or an N-oxide derivative thereof, in combination with a pharmaceutical vehicle or a suitable excipient.

Depending on the administration route selected, the daily dosage for a human being weighing 60 kg will be between 2 and 500 mg of active principle.

The compounds of formula I can be obtained:

I. When B represents a -S- or $-SO_2-$ group and A represents an alkylene radical, by condensing, in the presence of an acid acceptor and in a polar solvent such as dimethylsulphoxide or an alcohol, for example butanol, or a ketone such as methyl ethyl ketone, or a non-polar solvent such as an aromatic hydrocarbon, for example benzene, toluene or xylene, a 4-alkoxyphenyl derivative of general formula:

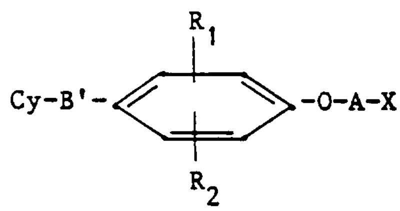

(2)

in which B' represents a -S- or $-SO_2-$ group, Cy, $R_1$ and $R_2$ have the same meaning as above, A represents am alkylene radical as defined in the formula (1) and X represents a halogen atom, preferably bromine, or an alkylsulphonyloxy group having from 1 to 4 carbon atoms such as for example, methanesulphonyloxy, or an arylsulphonyloxy group having from 6 to 10 carbon atoms, such as benzenesulphonyloxy or p-toluenesulphonyloxy, with an amine of general formula:

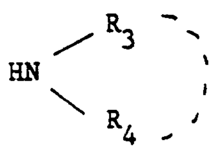

(3)

in which $R_3$ and $R_4$ have the same meaning as above to form the desired aminoalkoxyphenyl derivative of formula (1) in the form of a free base.

In general, the condensation in question is performed at a temperature between room-temperature and the refluxing-temperature of the medium, the acid acceptor being, for example, an alkali metal carbonate or hydroxide or an excess of amine of formula (3).

The compounds of formula (2) in question can be obtained:

a) when X is a halogen, by condensation of a 4-hydroxyphenyl derivative of general formula:

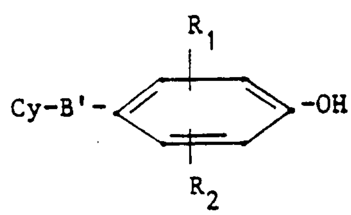

(4)

in which Cy, B', $R_1$ and $R_2$ have the same meaning as above, with a dihaloalkane of general formula

Hal-A-Hal (5)

in which A denotes an alkylene radical as defined in the formula (1) and Hal denotes a halogen atom, preferably bromine, this reaction being performed under reflux in a solvent such as methyl ethyl ketone or N,N-dimethylformamide and in the presence of a basic agent such as an alkali metal carbonate, for example potassium carbonate, an alkali metal hydride such as sodium hydride, an alkali metal hydroxide, for example sodium or potassium hydroxide, or an alkali metal alcoholate, for example sodium methylate or ethylate,

b) when X denotes am alkylsulphonyloxy or arylsulphonyloxy group, by condensation of a halide of general formula:

Hal-W

in which Hal has the same meaning as above and W denotes an alkylsulphonyl radical having from 1 to 4 carbon atoms, for example methanesulphonyl, or an arylsulphonyl radical having from 6 to 10 carbon atoms, for example benzenesulphonyl or p-toluenesulphonyl, in a solvent which is an acid acceptor, for example pyridine, with a 4-hydroxyalkoxy derivative of general formula:

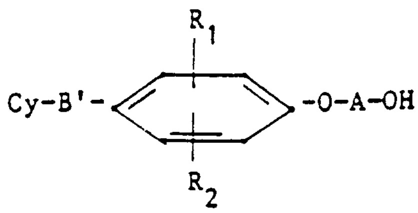

(6)

in which Cy, B', $R_1$ and $R_2$ have the same leaning as above and A denotes an alkylene radical as defined in formula (1).

As regards the compounds of formula (6), these can be prepared by condensing, in a suitable solvent such as N,N-dimethylformamide and in the presence of a basic agent such as an alkali metal carbonate, for example potassium carbonate, an alkali metal hydroxide such as sodium or potassium hydroxide, an alkali metal hydride such as sodium hydride or an alkali metal alcoholate, for example sodium methylate or ethylate, a 4-hydroxyphenyl derivative of formula (4) above with a halogenated alcohol of general formula:

Hal-A-OH (7)

in which A denotes an alkylene radical as defined in the formula (1) and Hal has the same meaning as above.

The compounds of formula (4) can be prepared in a general procedure, by adapting to the desired compound the method described in US patent No. 4,117,128 or the methods described hereunder.

In most cases, those compounds of formula (4) can be obtained by fixing a 4-O-protected benzenesulphonyl or phenylthio chain to the required carbocycle or heterocycle using a Friedel-Crafts reaction and deprotecting the oxygen in the 4-position of the benzenesulphonyl or phenylthio group by means of classical procedures to regenerate the OH group.

Hereunder are examples of methods commonly used for preparing derivatives of formula (4):

1) The compounds of formula (4) in which Cy represents a 2-R-indolizin-3-yl group can be prepared by reacting an indolizine derivative of general formula:

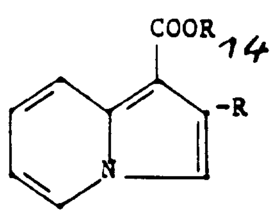

(8)

in which R has the same meaning as above and $R_{14}$ represents a lower alkyl radical preferably ethyl, with a halide of general formula:

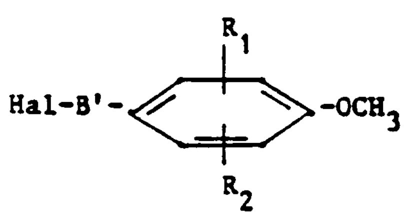

(9)

in which B', $R_1$, $R_2$ and Hal have the same meaning as above and in the presence of a Friedel-Crafts

catalyst such as aluminium chloride to provide a compound of general formula:

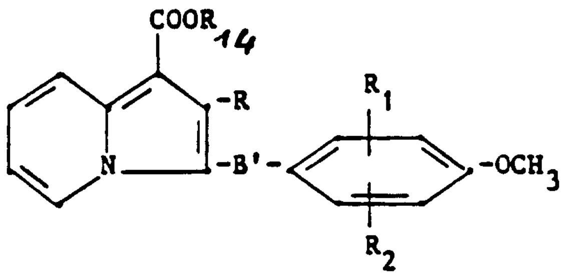

(10)

in which B', R, $R_1$, $R_2$ and $R_{14}$ have the same meaning as above.

The compound of formula (10) is subsequently demethylated using an ethanethiol/aluminium chloride mixture to give a 4-methoxyphenyl derivative of general formula:

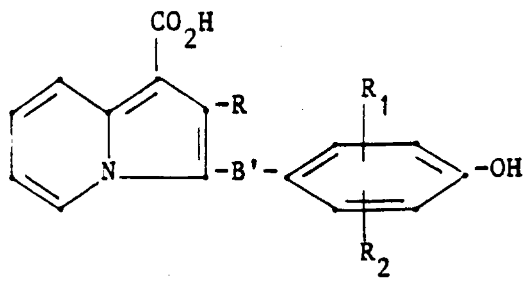

(11)

in which B', R, $R_1$ and $R_2$ have the same meaning as above which, when heated to about 200 °C provides the required compound of formula (4).

The compounds of formula (8) are either known compounds having been published in J. Chem. Soc. 1962 pp. 2627-2629 or compounds which can be prepared in accordance with the method described therein.

2) The compounds of formula (4) in which Cy represents a 2-R-quinolin-3-yl group can be prepared by reacting an $\alpha$-haloketone of general formula :

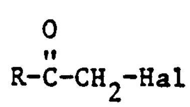

(16)

in which R and Hal have the same meaning as above, with a metal derivative of general formula :

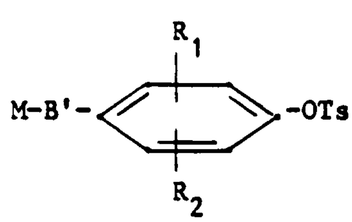

(17)

in which M, B', $R_1$ and $R_2$ have the same meaning as above and Ts represents a p-toluenesulphonyl group, to provide a ketone of general formula :

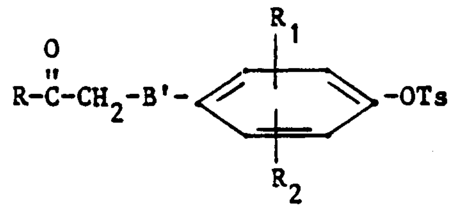

(18)

in which B', R, $R_1$ $R_2$ and Ts have the same meaning as above.

This ketone of formula (18) when treated with 2-amino-benzaldehyde [Helv. Chem. Act. vol. XVIII, p. 1235 (1935)] gives the 4-methoxyphenyl derivative of general formula :

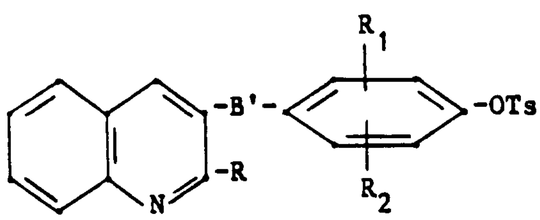

(19)

in which B', R, $R_1$, $R_2$ and Ts have the same meaning as above, which is subsequently hydrolysed in basic medium for instance in aqueous alkali metal hydroxide, to provide the required compound of formula (4).

3) The compounds of formula (4) in which Cy represents a 2-R-pyrazolo [1,5-a]pyrid-1-yl group can be prepared, in accordance with the method described in European patent application No. 121, 197, by treating a 2-R-pyrazolo[1,5-a]pyridine with a halide of formula (9) in the presence of a Friedel-Crafts catalyst such as for example aluminium chloride, to provide the 4-methoxyphenyl derivative of general formula:

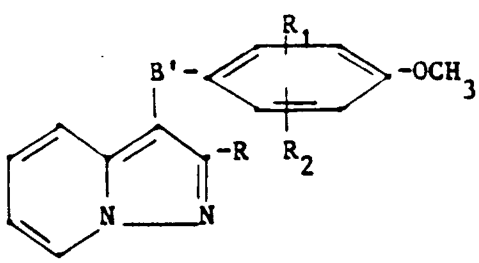

(24)

in which B', R, $R_1$ and $R_2$ have the same meaning as above.

The pyrrolopyridine derivative of formula (24) is then demethylated for instance by using pyridine hydrochloride at 200 - 220 °C to provide the required compound of formula (4).

4) The compounds of formula (4) in which Cy represents a phenyl group can be prepared by reacting benzene with a halide of formula (9) in the presence of a Friedel-Crafts catalyst such as aluminium chloride, to provide the required compound of formula (4).

5) The compounds of formula (4) in which Cy represents a 2-R-phenyl group can be prepared by treating a halide of general formula:

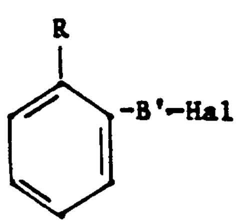

(25)

in which B', R and Hal have the same meaning as above, with a methoxyphenyl derivative of general formula:

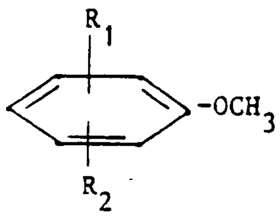

(26)

in which $R_1$ and $R_2$ have the same meaning as above, in the presence of a Friedel-Crafts catalyst such as aluminium chloride, to obtain the compounds of general formula :

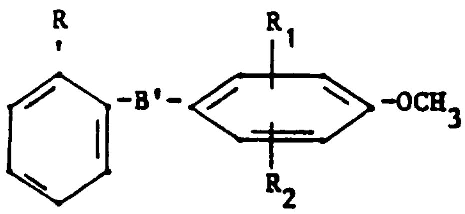

(27)

in which B', R, $R_1$ and $R_2$ have the same meaning as above

The compounds of formula (27) are then demethylated using for instance aqueous iodhydric acid to provide the required compound of formula (4).

Compounds of formula (25) are known products having been described in C.A. 81, 63285g, or can be obtained in accordance with known procedures.

Alternatively the compounds of formula (27) in which B' represents a $-SO_2-$ group can be prepared by treating the alkali metal derivative of a 2-R-benzenesulphonate, with a phenyl derivative of formula (26) in the presence of methanesulphonic acid/phosphorous pentoxide, in accordance with the method described in Communications, April 1984, p. 323.

6) The compounds of formula (4) in which Cy represents an optionally mono- or di-substituted 2-R-furan-3-yl group can be prepared starting from the corresponding mono- or di-substituted 2R-4,5-dihydrofuran-3-yl group which can be synthesized by heating a ketone derivative of formula (18) with a 1,2-dihalogenoethane of general formula :

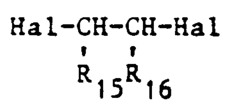

(28)

in which $R_{15}$ and $R_{16}$ which are the same or different, each represent hydrogen, a lower alkyl radical or a phenyl radical, in the presence of a basic agent such as an alkali metal carbonate, to obtain a cyclopropane derivative of general formula :

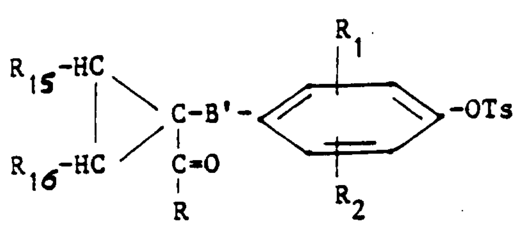

(29)

in which B', R, $R_1$, $R_2$, $R_{15}$, $R_{16}$ and Ts have the same meaning as above.

The cyclopropane derivative of formula (29) is subsequently heated between 100 and 130 °C in the presence of a phase transfer catalyst such as for instance triphenylphosphine or tricaprylylmethyl ammonium chloride to provide a 4-tosyloxyphenyl derivative of general formula:

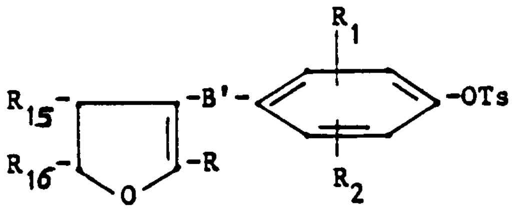

(30)

in which B', R, $R_1$, $R_2$, $R_{15}$, $R_{16}$ and Ts have the same meaning as above and the said 4-tosyloxyphenyl derivative is then detosylated by treatment with a basic agent such as an alkali metal hydroxide, to provide the required compound of formula (4).
The compounds of formula (4) in which Cy represents an optionally mono- or di- substituted 2-R-furan-3-yl group can be obtained by oxidizing for instance with manganese oxide, a 4,5-dihydrofuran derivative of formula (30) to obtain a furan derivative of general formula:

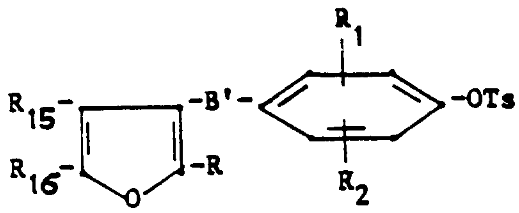

(31)

in which B', R, $R_1$, $R_2$, $R_{15}$, $R_{16}$ and Ts have the same meaning as above, which furan derivative is subsequently treated with a basic agent such as an alkali metal hydroxide, to obtain the required compound of formula (4).

Alternatively, The compounds of formula (4) in which Cy represents a 2-R-furan-3-yl can be prepared by reacting a compound of general formula:

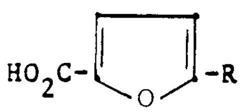

(32)

in which R has the same meaning as above with a halide of formula (9) and in the presence of a Friedel-Crafts catalyst such as aluminium chloride to obtain a 4-methoxy derivative of general formula:

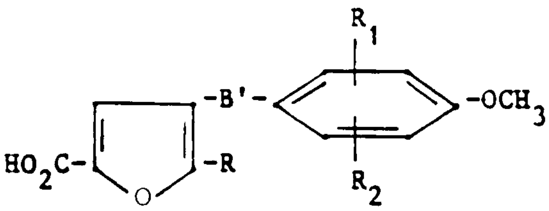

(33)

in which B', R, $R_1$, and $R_2$ have the same meaning as above, which is subsequently decarboxylated by heating and demethylated with an appropriate agent such as pyridine hydrochloride or aqueous hydrobromic acid, to provide the required compound of formula (4).

Alternatively, the compounds of formula (4) in which Cy represents an optionally substituted 2-R-furan-3-yl group can be prepared by oxidizing, for instance with manganese oxide, a sulphide

derivative of formula (30) to obtain am optionally substituted 2-R-3-(4-tosyloxybenzenesulphonyl)furan derivative which is subsequently treated by a basic medium for instance an alkali metal hydroxide, to provide the required compound of formula (4).

7) The compounds of formula (4) in which Cy represents a 1-R-benzimidazol-2-yl group can be obtained by reacting a 1-R-benzimidazole with a halide of formula (9) in the presence of a Friedel-Crafts catalyst such as aluminium chloride, to obtain a compound of general formula :

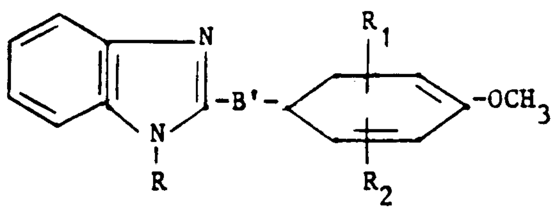

(34)

in which B', R, $R_1$ and $R_2$ have the same meaning as above, which is subsequently demethylated using an ethanethiol/aluminium chloride mixture or 2-mercaptoethanol in the presence of sodium hydride to obtain the required compound of formula (4).

Compounds of formula (34) in which $-OCH_3$ is replaced by -O Benzyl can also be used. In such case the compounds of formula (34) in question are debenzylated using for instance palladium charcoal for obtaining the required compound of formula (4).

When R represents hydrogen, benzimidazole is protected in the 1-position with an appropriate N-protecting group for instance a benzyl group which can subsequently be removed, if desired, using classical procedures.

8) The compounds of formula (4) in which Cy represents an optionally substituted 5-R-isoxazol-4-yl derivative can be prepared by reacting an isoxazole derivative of general formula :

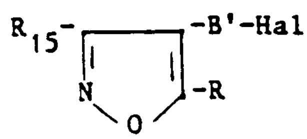

(35)

in which B', R, $R_{15}$ and Hal have the same meaning as above with a 4-methoxy derivative of formula (26) in the presence of a Friedel-Crafts catalyst such as aluminium chloride to obtain the compounds of general formula :

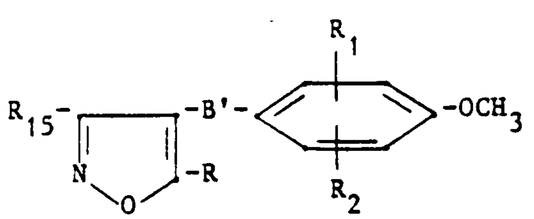

(36)

in which B', R, $R_1$, $R_2$ and $R_{15}$ have the same meaning as above, which is demethylated, using for instance aluminium chloride, to provide the required compound of formula (4).

Compounds of formula (35) are known products having been described in Gazz. Chim. Ital. 76, 30 (1946) while the other compounds of formula (35) can be obtained in accordance with the method described therein or classical methods.

Alternatively, the compounds of formula (36) in which $R_{15}$ represents hydrogen and B' represents a $-SO_2-$ group, can be obtained in accordance with the method described in J. Hetero. Chem. 23, 1363 (1986) by reacting a 1-(4-methoxy-benzenesulphonyl)-2-N,N-dimethylaminoethene with hydroxylamine.

Similarly, compounds of formula (36) in which B' represents a $-SO_2$-group, $R_{15}$ is other than hydrogen and in which $-OCH_3$ is replaced by -O) Tosyl can be used for obtaining the corresponding compounds of formula (4). These 3-substituted -5-R-3-(4-O-Tosyl)-benzenesulphonyl isoxazole derivatives can be prepared in accordance with the method described in Gazz. Chim. Ital. 98, 656 (1968) i.e. by reacting a benzenesulphonyl-ketone and an hydroxamic acid derivative.

9) The compounds of formula (4) in which Cy represents a 1-$R_{11}$-2-R-indol-3-yl or 1-$R_{11}$-3-R-indol-2-yl derivative can be prepared :

a) when $R_{11}$ represents hydrogen, by reacting p-methoxythiophenol substituted by $R_1$ and $R_2$ groups, with 2-R-indole or 3-R-indole in the presence of iodine, to provide an indole derivative of general formula :

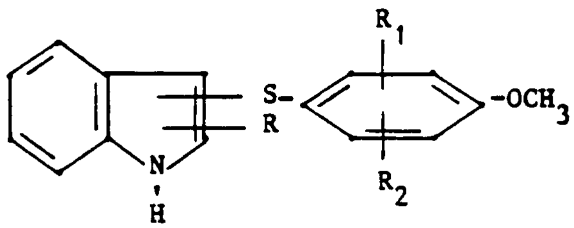

(39)

in which R, $R_1$ and $R_2$ have the same meaning as above, which can then be oxidized with 3-chloroperbenzoic acid to provide the sulphonyl derivatives of general formula :

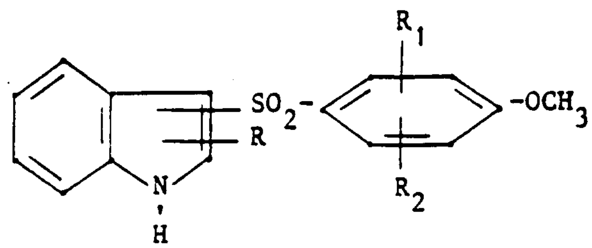

(40)

in which R, $R_1$ and $R_2$ have the same meaning as above.

The compounds of formulae (39) and (40) can subsequently be demethylated using 2-mercaptoethanol in the presence of sodium hydride to provide the required compounds of formula (4).

b) when $R_{11}$ is other than hydrogen, by treating a compound of formula (39) or (40) with an iodide of formula $R_{11}$-I in which $R_{11}$ is other than hydrogen and demethylating the 1-substituted derivative so obtained with 2-mercaptoethanol in the presence of sodium hydride, to provide the required compounds of formula (4).

in which B', R, $R_1$, $R_2$ and $R_{11}$ have the same meaning as above, to provide the required compounds of formula (4).

The compounds of formula (44) can be obtained in accordance with the method described in Tetrahedron Lett. 23, pp. 2373-2374 (1972) i.e. from a sulphonylmethylisocyanide and an imidazol derivative.

10) The compounds of formula (4) in which Cy represents 2-R-benzimidazol-1-yl group can be obtained by reacting a 2-R-benzimidazole with a halide of formula (9) in the presence of a Friedel-Crafts catalyst such as aluminium chloride, to provide a compound of general formula :

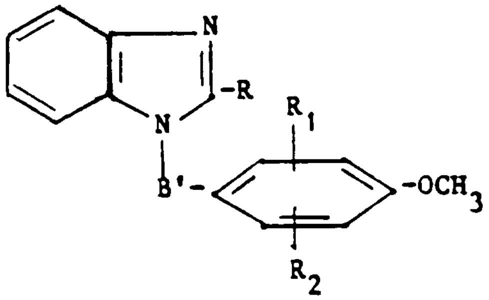

(47)

in which B', R, $R_1$ and $R_2$ have the same meaning as above

The compound of formula (47) is then demethylated using an appropriate agent for instance an ethanethiol/aluminium chloride mixture to give the required compound of formula (4).

According to an alternative method, the compounds of formula (1) in which B represents a -S- or $-SO_2-$ group and A represents an alkylene radical, preferably those in which A represents a propylene radical, can also be obtained by reacting, in the presence of a basic agent such as an alkali metal carbonate, for example potassium carbonate, an alkali metal hydroxide such as sodium or potassium hydroxide, an alkali metal hydride such as sodium hydride or an alkali metal alcoholate, for example sodium methylate or ethylate, a 4-hydroxyphenyl derivative of formula (4) above with a compound of general formula:

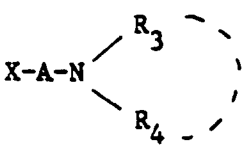

(52)

in which X has the same meaning as above and preferably represents chlorine or a benzenesulphonyloxy or p-toluenesulphonyloxy radical, A represents an alkylene radical and $R_3$ and $R_4$ have the same meaning as above, the reaction taking place at a temperature between room-temperature and the refluxing temperature of the medium and in a polar solvent such as methyl ethyl ketone or dimethylsulphoxide to form the desired aminoalkoxyphenyl derivative of formula (1) in the form of the free base.

When $R_4$ represents hydrogen, the nitrogen atom is preferably protected by a labile group for instance a protecting group which can be eliminated in basic medium for example the tertiobutoxycarbonyl (BOC) group.

The compounds of formula (52) are products which are known or which can be prepared by known methods.

The compounds of formula (1) in which Cy represents a 2-R-benzimidazole, A represents an alkylene chain and B represents a -S- or $-SO_2-$ group can also be prepared by reacting 2-R-benzimidazole with a halide of general formula:

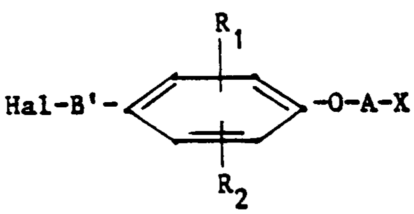

(53)

in which B', $R_1$, $R_2$, Hal and X have the same meaning as above and A represents an alkylene chain, in the presence of an acid acceptor such as triethylamine to obtain a compound of general formula:

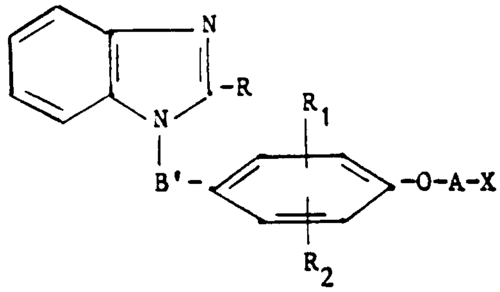

(54)

in which B', R, $R_1$, $R_2$ and X have the same meaning as above, and A represents an alkylene chain, which compound is subsequently reacted with an amine of formula (3) to obtain the required compound of formula (1) in the form of a free base.

Similarly, the compounds of formula (1) in which Cy represents an optionally mono- or di- substituted 2-R-4,5-dihydro-furan-3-yl group, A represents an alkylene chain and B represents a -S- or $SO_2$- group, can be prepared by hydrolysing a cyclopropane derivative of formula (29) in the presence of an aqueous alkali metal hydroxide solution to provide a 4-methoxyphenyl derivative of general formula:

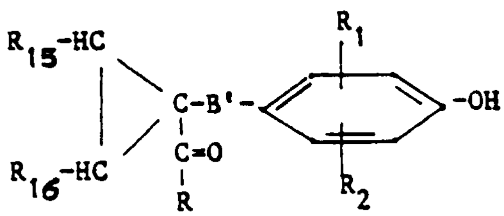

(55)

in which B', R, $R_1$, $R_2$, $R_{15}$ and $R_{16}$ have the same meaning as above, which is then reacted:

- with a dihaloalkane of formula (5) and the resulting product with an amine of formula (3)
  or
- with a compound of general formula (52) , to provide an aminoalkoxyphenyl derivative of general formula:

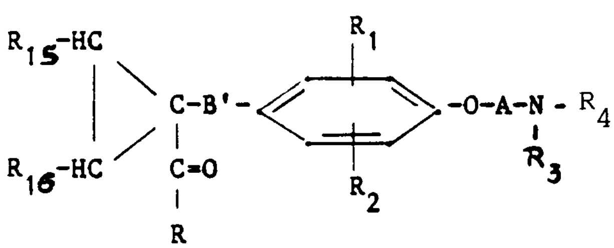

(56)

in which B', R, $R_1$, $R_2$, $R_3$, $R_4$, $R_{15}$ and $R_{16}$ have the same meaning as above and A represents an alkylene chain.

The cyclopropane derivative of formula (56) is subsequently heated between 100 and 130 °C in the presence of a phase transfer catalyst such as for instance triphenylphosphine or tricaprylylmethyl ammonium chloride to provide the required 2,3-dihydrofuran derivative of formula (1) in the form of a free base.

II. When B represents a -SO- group, by treating, with an oxidizing agent, a sulphide of formula (1) in which a represents a -S- group, this compound of formula (1) being in the form of the free base of a salt thereof so as to obtain the required compound in the form of the free base or a salt thereof.

Where the required compound is provided in the form of a salt, the free base thereof can be recovered by treatment with a basic agent such as an alkali metal carbonate for example potassium carbonate or an alkali metal bicarbonate for example sodium bicarbonate.

Generally, the reaction takes place in water or in an organic solvent such as methylene chloride and in the presence of a suitable oxidizing agent such as for example sodium periodate, potassium

permanganate or 3-chloroperbenzoic acid.

Depending on the oxidizing agent used, mixtures of sulphoxides or sulphones can be obtained. These mixtures can be separated by conventional procedures for instance by chromatography.

III. When B represents a -S- or $-SO_2-$ group and A represents an optionally substituted 2-hydroxypropylene chain, by reacting under reflux a 4-hydroxyphenyl derivative of formula (4) with an epihalohydrin, such as epichlorhydrin or epibromhydrin in dextrorotatory or laevorotatory form or in the form of a mixture of these isomers, for example in racemic form, and in the presence of a basic agent such as an alkali metal carbonate, for example potassium carbonate, an alkali metal hydroxyde, for example sodium or potassium hydroxide, an alkali metal hydride, such as sodium hydride or an alkali metal alcoholate, for example sodium methylate of ethylate, and in a polar solvent such as methyl ethyl ketone to give the oxiranylmethoxy derivatives of general formula :

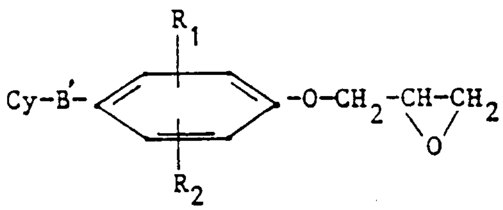

(57)

in which Cy, B', $R_1$ and $R_2$ have the same meaning as above.

The oxyranylmethoxy derivatives of formula (57) are then treated under reflux with an amine of formula (3), this being performed in a polar solvent such as methyl ethyl ketone or in an excess of amine of formula (3) to give the desired compound of formula (1) in the form of the free base in which A represents a 2-hydroxypropylene chain which can be reacted, if desired, with a lower alkyl halide in the presence of a strong base to provide the compound of formula (1) in the form of the free base in which A represents a 2-hydroxypropylene chain in which the hydroxy is substituted by a lower alkyl radical.

In some cases, by-products may be formed in parallel with the compounds of formula (57) above, on this case 4-(3-halo-2-hydroxypropoxy)benzenesulphonyl derivatives.

On reaction with the amine of formula (3), these derivatives will nevertheless give rise to the desired compounds of formula (1) in which A represents a 2-hydroxypropylene chain.

The compounds of formula (1) thereby obtained in the form of the free base can then be converted to pharmaceutically acceptable salts by reaction with a suitable organic or inorganic acid, for example oxalic, maleic fumaric, methanesulphonic, benzoic, ascorbic, pamoic, succinic, hexamic, bismethylenesalicylic, ethanedisulphonic, acetic, propionic, tartaric, salicylic, citric, gluconic, lactic, malic, cinnamic, mandelic, citraconic, aspartic, palmitic, stearic, itaconic, glycolic, p-aminobenzoic, glutamic, benzenesulphonic or theophyllineacetic acid or with lysine or histidine.

Similarly, the N-oxide derivatives of the compounds of formula (1) can be formed by oxidizing the compound of formula (1) in question with an appropriate oxidizing agent for instance hydrogen peroxide or 3-chloroperbenzoic acid.

C.I. Sharpe describes in Journal of medicinal Chemistry (1972) vol. 15, No. 5, p. 523 a diethylaminoalkoxybenzenesulfid-benzothiazole which exhibits antidepressive properties.

Monoalkyl- or dialkylaminoalkoxybenzenesulphonyl-benzofuran or benzothiophene derivatives are reported in US patent No. 4, 117, 128 as presenting pharmacological effects in the cardiovascular field.

In the course of the elaboration of the present invention, tests were carried out with compounds specifically cited in the aforesaid US patent, more particularly with 2-ethyl- or 2-n-butyl-1-3-[4-(2-diethylaminoethoxy)benzenesulphonyl]benzofuran.

From results of these tests, it could be concluded that in the dog at the dose of 10 mg/kg by intravenous route, these known compounds only present a weak $\alpha$-antiadrenergic activity and no or practically no $\beta$-antiadrenergic effect.

Similarly indolizine derivatives which are substituted in the 1-position with an alkyloxybenzoyl chain which is itself substituted with a mono- or di-alkyl amino group, and which are stated to have pharmacological effects in the cardiovascular field, are already known.

In this connection, there may be mentioned French Patent No. 2,341,578 and Eur. J. Med. Chem. 1977, 12, No. 4 pp. 345-350, which specifically describe 2-ethyl-, 2-n-propyl- or 2-n-butyl-1-[4-(3-di-n-propyl- or 3-di-n-butylaminopropoxy)benzoyl]indolizine optionally dimethylated on the benzoyl radical.

These known compounds showed antiadrenergic activities which were non-existent or low, at all events too low to be of any value for therapy.

Other monoalkyl- or dialkyl-aminoalkoxybenzoyl derivatives are also known.

For instance are reported in the literature monoalkyl- or dialkyl-aminobenzoyl derivatives of :

- thiophene [(J. Med. Chem. V, 13 (3) pp. 359-366 (1970)]
- naphthalene or dihydronaphthalene (Chim. Ther. V, 7 (5) pp. 369-377)
- pyridine [Ing. Chim. V, 59 (283) pp. 3-13 (1977)]
- thieno[3,2-c]pyridine [Heterocycles, V, 22 (5), pp. 1235-1247 (1984)]
- indole [Eur. J. Med. Chem. - Chim. Ther. V, 12 (5) pp. 483-487 (1977)]
- furan (French patent No. 2,400,515)
- chromone (US patent No. 4,220,645).

Tests carried out with these known compounds showed that same of them presented antiadrenergic activities but which were low, at all events too low to be of any value for therapy.

It has now been found, in addition, that mono- or di-alkylaminoalkoxy benzenesulphonyl derivatives ressembling those described in U.S. patent No. 4,117,128 but in which the benzofuran or benzothiophene moiety has been replaced by another carbocyclic or heterocyclic ring present more valuable antiadrenergic properties than known sulphonyl derivative: of the aforesaid U.S. patent or known benzoyl derivatives of the above-cited references.

For instance, $\alpha$- and $\beta$-antiadrenergic properties were registered, in the dog by intravenous route at doses as low as 0.1 to 1.5 mg/kg with respect to mono- or di-alkylaminoalkoxybenzenasulphonyl derivatives of formula (1) in which Cy represents another group than benzofuryl or benzothienyl for instance a quinolinyl or pyrrolo[1,2-b]pyridazinyl moiety.

A particularly valuable class of compounds of the invention are those in which $R_1$, $R_2$, $R_4$ and A have the same meaning as in formule , B represents a $-SO_2-$ group, $R_3$ represents an alkyl radical and Cy represents a group selected from :

- quinolinyl more particularly 2-R-quinolin-3-yl
- pyrrolo[1,2-b]pyridazinyl more particularly 6-R-pyrrolo[1,2-b] pyridazin-5-yl.
- pyrazolo[1,5-a]pyridyl more particularly 2-R-pyrazolo[1,5-a]pyrid-3-yl.
- imidazo[1,2-a]pyridyl more particularly 2-R-imidazo[1,2-a]pyrid-3-yl.
- 4,5-dihydrofurannyl more particularly 2-R-4,5-dihydrofuran-3-yl.
- indolyl more particularly 2-R-indol-3-yl and 1-$R_{11}$-2-R-indol-3-yl.
- indolizin-3-yl.

Moreover, it has been found that the calcium inhibitory activity of the compounds of the invention is at least equal to, if not greater than, that observed in tests performed with the known compounds. In contrast to the known compounds, it has thus been possible to demonstrate for the compounds of the present invention a pharmacological spectrum revealing anticalcium and $\alpha$- and $\beta$-antiadrenergic components with a balanced intensity which is of therapeutic value, for example, for treatment of angina.

As has been reported in detail by R. Charlier in "Bruxelles Medical", No. 9, September 1969, pages 543-560, it is accepted than an antianginal drug treatment should be capable, in particular, of antagonizing the antiadrenergic type cardiovascular reactions. To this end, agents capable of blocking the $\alpha$-receptors nave been proposed.

However, the clinical application of such compounds to the treatment of angina remained unsuccessful, very probably due to the fact that $\alpha$-receptor antagonists induce only a vary partial neutralization of the adrenergic system, the activity of the $\beta$-receptors being unaffected.

In fact, the most undesirable haemodynamic manifestations which occur in angina pectoris patients during their painful attacks are, most of all, cardiac, and consequently involve the $\beta$-receptors.

In parallel, treatments have been proposed with drugs which are $\beta$-adrenergic receptor antagonists. Those compounds, which are of genuine clinical value, decrease the attacks of angina by reducing the work of the heart by slowing the heart rate. However, there is no fall in the peripheral arterial resistance which, on the contrary, rises through release of the $\alpha$-tonicity.

These drug treatments nevertheless modify some haemodynamic parameters in a direction which, at a fundamental level, detracts from the value of these drugs for angina pectoris patients in particular and heart patients in general.

If the antiadrenergic aspect of $\beta$-blockers is considered, it becomes clear that only the tachycardia and the increase in the force and the speed of contraction of the heart are capable of being neutralized, the arterial hypertension involving a stimulation of the $\alpha$-receptors on which $\beta$-antagonists have no action.

In fact, while the cardiovascular disturbances brought about by the stimulation of the $\beta$-receptors are the more harmful to angina patients, it is nonetheless true that arterial hypertension also plays a not insignificant part.

In addition, blocking the β-receptors involves a risk, depriving the patient suffering from cardiac insufficiency of a compensatory mechanism which he normally brings into play to limit his circulatory insufficiency.

This reflex mechanism, the main component of which makes use of the pathway of the β-adrenergic system, leads, in particular, to an increase in the force and the speed of contraction of the heart. In consequence, if this system is blocked, the patient suffering from cardiac insufficiency experiences a worsening of his functional breakdown. It is hence logical to consider that the use of a β-blocker whose action is pure and complete will always involve a cardiac risk.

It hence appears to be desirable not to seek complete α- or β-antagonistic properties, given the clinical side effects that these can bring about. It seems more logical to aim to subdue rather than to eliminate the cardiovascular disturbances which characterize the hyperstimulation of the adrenergic system as a whole.

The compounds of the invention meet this objective since they show incomplete α- and β-type antiadrenergic properties. They can hence be considered, not as β-blockers but as adreno-decelerators, that is to say partial antagonists of the α- and β-adrenergic reactions, potentially deviod if the disadvantages listed above for β-blockers.

In addition, the calcium inhibitory component demonstrated in the compounds of the invention will act as an exceptional complement to the pharmacological spectrum of their cardiovascular action.

It is known, in effect, that the transport of calcium ions is one of the main components of the action potential in heart cell and, in consequence, this transport plays a fundamental part in the electrical conduction as well as in the disorders which may occur therein (arrhythmia). In addition, it is known that calcium ions are involved in the excitation-contraction coupling which controls the degree of vasoconstriction in smooth muscle and, in the same circumstances, plays a critical part in attacks of angina pectoris.

Compounds which are calcium antagonists act at the level of the cell membrane by selectively preventing calcium from participating in the process of contraction within the arterial cell.

In fact, it appears increasingly clear, at the present time, that the clinical results provided by the combination of calcium inhibitors and β-adrenergic inhibitors are better than when each inhibitor is used separately (J.A.M.A. 1982, 247, pages 1911-1917).

It appears, moreover, that no β-blocker which exerts, in addition, a significant inhibitory action in respect of calcium transport exists at the present time.

From this standpoint, the compounds of the invention possessing both an anticalcium component and an α- and β-antiadrenergic component will be of fundamental value, since they are capable of more extensive therapeutic applications than a separate β-blocker or a separate calcium inhibitor.

Compounds of the invention possess an α- and β-antiadrenergic component reinforced by an oxygen-economizing effect capable of providing a therapeutic effect in man in the syndrome of angina of effort, which can, moreover, be treated by traditional β-blockers. However, the major advantage of these compounds will reside in the fact that they may, as a result of their anti-calcium effect, be used in the treatment of angina at rest, a syndrome induced by the appearance of a spasm in the coronary arteries, which is combated at present by compounds such as diltiazem, verapamil or nifedipine.

In addition, compounds of the invention were also shown to be capable of inducing a substantial increase in the coronary flow.

The results of pharmacological tests performed for the purpose of determining the cardiovascular properties of the compounds of the invention are recorded below.

I. Calcium inhibitory properties

The properties of inhibiting calcium transport at the membrane level shown by the compounds of the invention were demonstrated by measuring their antagonistic action with respect to the contractile response to potassium-induced depolarization on isolated rat aorta. It is well established that the depolarization of a smooth muscle membrane by potassium renders the latter permeable to extracellular calcium and induces muscle contraction.

Consequently, the measurement of the inhibition of the contractile response to depolarization by potassium, or the measurement of a relaxation of the tonic contraction on potassium depolarization, can represent an evaluation of the power of a compound as an inhibitor of the membrane permeability to $Ca^{++}$ ions.

The technique used was as follows:
The aorta was removed from male Wistar rats weighing approximately 300g, and cut into strips approximately 40 mm long and 3 mm wide.

These fragments were placed in a 25-ml isolated organ trough containing a modified Krebs-bicarbonate solution (112 mM NaCl; 5 mM KCl; 25 mM $NaHCO_3$; 1 mM $KH_2PO_4$; 1.2 mM $MgSO_4$; 2.5 mM $CaCl_2$; 11.5 mM glucose, distilled water to 1000ml) through which a stream of 5-7% carbon dioxide in oxygen was passed, and maintained at 37°C. The preparation was connected to a force microsensor and the contractile response recorded after amplification on a recorder.

A tension of 2g was applied to the preparation. This tension was maintained for 60 minutes in the modified Krebs-bicarbonate solution, and contractions were then induced by replacing the Krebs-bicarbonate solution by a potassium-Krebs solution (17 mM NaCl; 100 mM KCl; 25 mM $NaHCO_3$; 1 mM $KH_2PO_4$; 1.2 mM $MgSO_4$; 2.5 mM $CaCl_2$; 11.5 mM glucose; distilled water to 1000ml). When the contractile response of the preparation had become reproducible, a given amount of a compound of the invention was introduced into the bath. Sixty minutes later, a new spasm was induced by potassium depolarization.

The results obtained on the aorta used in the experiment were then expressed as a percentage of the maximum contractional effect before incubation with the test substance.

By way of examples, the results which follow were obtained, the compounds of formula (1) being in the form of the base, hydrochloride or oxalate.

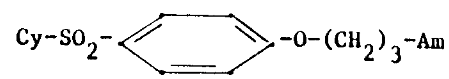

| Compound | Cy | Am | % of the maximum contractional effect | | | |
|---|---|---|---|---|---|---|
| | | | $10^{-6}M$ | $10^{-7}M$ | $10^{-8}M$ | $10^{-9}M$ |
| Ex. 11 | 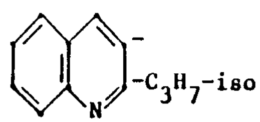 | $-N(n-C_4H_9)_2$ | 22.4 | 67.3 | - | - |
| Ex. 9 | 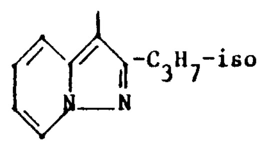 | $-N(n-C_4H_9)_2$ | 0 | 18.9 | 83 | - |

| | | | | | | |
|---|---|---|---|---|---|---|
| Ex. 10 | 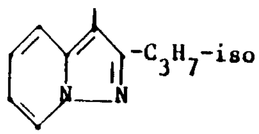 | $-NH-C(CH_3)_3$ | 13.2 | 66.5 | 84 | – |
| Ex. 7 | 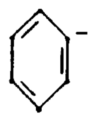 | $-N(n-C_4H_9)_2$ | 32.4 | 85.3 | – | – |
| Ex. 8 | 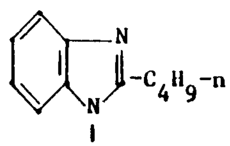 | $-N(n-C_4H_9)_2$ | 47.5 | 64.8 | – | – |

| | | | | | | |
|---|---|---|---|---|---|---|
| Ex. 13 | 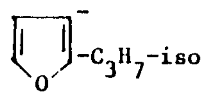 | $-N(n-C_4H_9)_2$ | 4.0 | 59.4 | 90.3 | - |
| Ex. 14 | 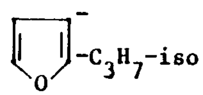 | $-NH-C(CH_3)_3$ | 10.3 | 77.9 | 94.3 | - |

| | | | | | | | |
|---|---|---|---|---|---|---|---|
| Ex. 19 | 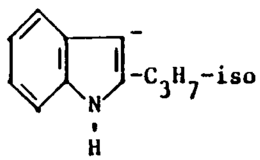 | $-N-(n-C_4H_9)_2$ | 4.9 | 37.4 | 66.0 | - | |
| Ex. 20 | 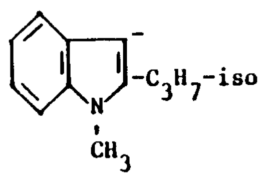 | $-N-(n-C_4H_9)_2$ | 0 | 15.1 | 71.0 | 84.6 | |

| | | | | | | |
|---|---|---|---|---|---|---|
| Ex. 22 | 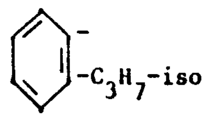 | $-N-(n-C_4H_9)_2$ | - | 43.2 | - | 89.1 |
| Ex. 23 | 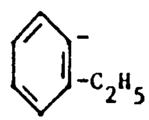 | $-N-(n-C_4H_9)_2$ | 4.2 | 43.4 | 85.1 | - |
| Ex. 17 | 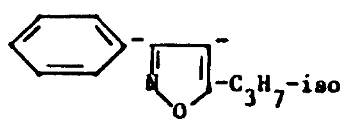 | $-N-(n-C_4H_9)_2$ | 10.0 | 38.7 | 75.8 | - |

| | | | | | | |
|---|---|---|---|---|---|---|
| Ex. 21 | 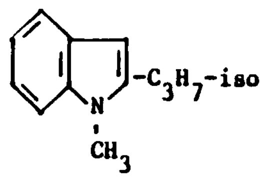 | $-NHC-(CH_3)_3$ | 8.1 | 51.7 | 84.5 | - |
| Ex. 2 | 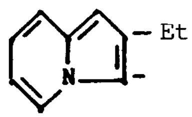 | $-N-(n-C_4H_9)_2$ | 5.8 | 16.8 | 54.6 | 75.3 |
| Ex. 1 | 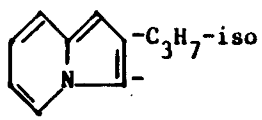 | $-NH-C-(CH_3)_3$ | 5.4 | 28.1 | 76.1 | 92.5 |
| Ex. 24 | 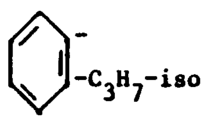 | $-NH-C-(CH_3)_3$ | 2.7 | 52.2 | 92.3 | - |

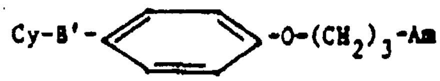

| Comp. | Cy | B' | Am | % of the maximum contractional effect | | |
|---|---|---|---|---|---|---|
| | | | | $10^{-5}M$ | $10^{-6}M$ | $10^{-7}M$ |
| Ex. 12 | 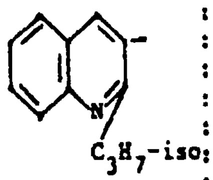 | $-SO_2-$ | $-NHC(CH_3)_3$ | - | 26.9 | 79.6 |
| Ex. 25 | 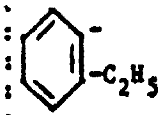 | $-SO_2-$ | $-NHC(CH_3)_3$ | - | 32.0 | 83.3 |

By way of comparison, the following results were obtained with known compounds :

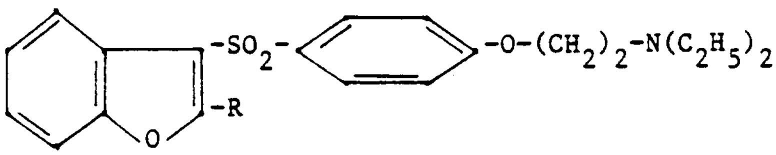

| Compound | R | % of the maximum contractional effect | | |
|---|---|---|---|---|
| | | $10^{-6}M$ | $10^{-7}M$ | $10^{-8}M$ |
| A | $n-C_4H_9-$ | 25 | 60.3 | 84.3 |
| B | $-C_2H_5$ | 52.2 | 84.9 | - |

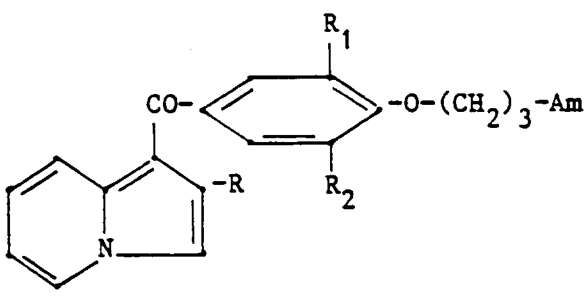

| Compound | R | $R_1$ | $R_2$ | Am | % of the maximum contractional effect | |
|---|---|---|---|---|---|---|
| | | | | | $10^{-6}$ M | $10^{-7}$ M |
| Compound C | $n-C_4H_9$ | H | H | $-N(n-C_4H_9)_2$ | 25.0 | 74.4 |
| Compound D | $-C_2H_5$ | $CH_3$ | $CH_3$ | $-N(n-C_4H_9)_2$ | 19.3 | 64.7 |
| Compound E | $-C_2H_5$ | H | H | $-N(n-C_3H_7)_2$ | 37.9 | 89.1 |

II. Antiadrenergic properties

The object of this test is to determine the capacity of the compounds of the invention for reducing the increase in epinephrine-induced increase in blood-pressure (anti-$\alpha$ effect) and the isoprenaline-induced acceleration in heart rate (anti-$\beta$ effect), in dogs previously anaesthatized with pentobarbital and atropinized.

For each dog, the dose of epinephrine (between 3 and 10 $\mu$g/kg) which induced a reproducible increase in the blood-pressure of approximately 133 x $10^2$ Pa and the dose of isoprenaline (1 to 2 $\mu$g/kg) which induced a reproducible increase in the heart rate of approximately 70 beats/min. were first determined. The dose of epinephrine and of isoprenaline, determined in this manner, were injected alternately every ten minutes and, after two successive reference responses had been obtained, an amount of the test compound was administered intravenously.

Anti-$\alpha$ effect

The percentage reduction in the hypertension induced by the test compound compared with the reference hypertension previously obtained (approximately 100 mm Hg) was recorded.

Anti-$\beta$ effect

The percentage reduction in the acceleration of the heart rate induced by the test compound compared with the reference tachycardia measured previously (approximately 70 beats) was recorded.

In both cases, the results of the reduction in blood-pressure or in the heart rate have been expressed as follows :

+ for a reduction < 50%
+ + for a reduction ≧ 50%
+ + + for a reduction sub-total (almost complete reduction).

The following results were recorded :

| Compound | Dose (mg/kg) | anti-$\alpha$ effect | anti-$\beta$ effect |
|---|---|---|---|
| Ex. 7 | 4.9 | + | + + |
| Ex. 8 | 5.89 | + + | + + |
| Ex. 9 | 0.12 | + + | + |
| Ex. 10 | 0.52 | + + | + |
| Ex. 11 | 1.2 | + + + | + |
| Ex. 13 | 3 | + + + | + + |

By way of comparison, the known compounds showed the following anti-adrenergic effects :

| Compound | Dose (mg/kg) | anti-α effect | anti-β effect |
|---|---|---|---|
| Compound A | 10 | + | 0 |
| Compound B | 10 | + + + | + |
| Compound C | 10 | + | 0 |
| Compound D | 10 | + | + |
| Compound E | 10 | + | + + |
| Compound F * | 10 | - | 0 |

* = 2-ethyl-1-{4-[3-(di-n-butylamino-propyloxy]benzoyl}-indolizine.

These results demonstrate that the compounds of the invention show much greater α- and β-antiadrenergic activity than those of the compounds of the prior art.

The therapeutic compositions according to the invention can be presented in any form suitable for administration in human or veterinary therapy. As regards the administration unit, this can take the form of, for example, a coated- or uncoated tablet, hard- or soft-gelatin capsule, packaged powder, suspension or syrup for oral administration, a suppository for rectal administration or a solution or suspension for parenteral administration.

The therapeutic compositions of the invention may contain, per administration unit, for example, from 50 to 500 mg as the weight of active ingredient for oral administration, from 50 to 200 mg of active ingredient for rectal administration and from 50 to 150 mg of active ingredient for parenteral administration.

Depending on the administration route chosen, the therapeutical veterinary compositions of the invention will be prepared by combining at least one of the compounds of formula I, or a non-toxic addition salt of this compound, with a suitable excipient, it being possible for the latter to consist, for example, of at least one ingredient selected from the following substances: lactose, starches, talc, magnesium stearate, polyvinylpyrrolidone, alginic acid, colloidal silica, distilled water, benzyl alcohol or sweetening agents.

The following non-limiting examples illustrate the invention:

EXAMPLE 5

Preparation of 2-isopropyl-3-{4-[3-(tert-butylamino)propyloxy]benzenesulphonyl} indolizine hydrochloride (SR 33712A)

a) 1-Ethoxycarbonyl-2-isopropyl-3-(4-methoxybenzenesulphonyl) indolizine

Into 114 ml of 1,2-dichloroethane were dissolved 13.4 g (0.058 mol) of 1-carboethoxy-2-isopropylindolizine and 12.7 g (0.061 mol) of 4-methoxybenzenesulphonyl chloride. The solution was stirred and cooled to 0 °C while 23 g (0.174 mol) of aluminium chloride were added by small fractions.

The addition was terminated after 30 min and the medium was allowed to return to room-temperature for 4 hours. After that, the mixture was poured onto ice and 20 ml of concentrated hydrochloric acid were added. The medium was stirred for 30 min and the organic layer was decanted and washed with 3 fractions of water. The extract was dried on sodium sulphate and isolated under vacuum to obtain 24.8 g of a black oil (theory : 23.28 g). This oil was purified on a silica column using first n-hexane/10%-ethyl acetate and then n-hexane/20%-ethyl acetate as eluents.

In this manner 3.25 g of 1-ethoxycarbonyl 2-isopropyl-3-(4-methoxybenzenesulphonyl) indolizine were obtained in a form of a white solid.
Yield : 13.95%.
M.P. 103-104 °C (hexane/methylene chloride).

b) 1-Carboxy-2-isopropyl-3-(4-hydroxybenzenesulphonyl) indolizine

Into 100 ml of methylene chloride and 25 ml of ethanethiol, were suspended 6.7 g (0.050 mol) of aluminium chloride. The suspension was stirred and cooled to 0 °C while 2.5 g of 1-ethoxycarbonyl-2-isopropyl-3-(4-methoxybenzenesulphonyl) indolizine in methylene chloride were added.The addition took about 15 min . The reaction medium was allowed to return to room-temperature and maintained, at this temperature, for 45 min . After pouring onto ice, 5 ml of concentrated hydrochloric acid were added while stirring and the medium was extracted with 2 fractions of ethyl ether. The ethereal extracts were collected

and washed with 3 fractions of 30ml of a 10%-aqueous solution of sodium carbonate. The aqueous phase was acidified and a precipitate was observed.

In this manner, 1g of crude 1-carboxy-2-isopropyl-3-(4-hydroxybenzenesulphonyl)indolizine was obtained in the form of a beige solid.
Yield : 44.6%

c) 2-Isopropyl-3-(4-hydroxybenzenesulphonyl)indolizine

For 2 min., 1g (2.78 x $10^{-3}$ mol) of 1-carboxy-2-isopropyl-3-(4-hydroxybenzenesulphonyl)indolizine was heated at 200 °C. The black residue so obtained was taken up in methylene chloride and a slight precipitate was eliminated by filtration. The filtrate was evaporated to provide 0.8g of a brown oil (theory : 0.877g). This oil was purified on a silica column using a methylene chloride/ethyl acetate 95/5 mixture as eluent and 0.6g of a green oil was isolated.

In this manner, 2-isopropyl-3-(4-hydroxybenzenesulphonyl)indolizine was obtained.
Yield : 68.4%
Purity : 97.5%

d) 2-Isopropyl-3-[4-(3-bromopropyloxy)benzenesulphony]indolizine

While stirring 1.6g (5.07 x $10^{-3}$ mol) of 2-isopropyl-3-(4-hydroxy-benzenesulphonyl)indolizine, 0.8g (5.6 x $10^{-3}$ mol) of potassium carbonate and 4.1g (20.2 x $10^{-3}$ mol) of 1,3-dibromopropane are slightly refluxed in 30ml of methyl-ethyl-ketone. After 24 hours, the medium was cooled and filtered to eliminate the mineral salts and the filtrate was isolated under vaccum and then under high vacuum to eliminate the 1,3-dibromopropane in excess.
The oily residue was taken up in water containing sodium hydroxide and the brominated product was extracted to provide 1g of a compound which was purified on a silica column using dichloromethane as eluent.

In this manner, 0.9g of 2-isopropyl-3-[4-(3-bromopropyloxy) benzenesulphonyl]indolizine was obtained in the form of a greenish oil which solidified after some days.
Yield : 40.67%

e) 2-Isopropyl-3-{4-[3-(tert-butylamino)propyloxy]benzenesulphonyl} indolizine hydrochloride

A mixture of 0.9g (2.06 x $10^{-3}$ mol) of 2-isopropyl-3-[4-(3-bromo-propyloxy)benzenesulfonyl]indolizine and 0.76g (10.3 x $10^{-3}$ mol) of tert-butylamine in 4ml of dimethylsulphoxide are stirred for 48 hours at room-temperature. The dimethylsulphoxide was eliminated under vaccum together with the tert-butylamine in excess and the residue was taken up in water containing sodium hydroxide.
The medium was then extracted with dichloromethane to give 0.8g of a yellow oil which was purified on an alumina column using an ethyl acetate/methanol 95/5 mixture as eluent. The oil so obtained (0.65g) was then transformed into the hydrochloride which was recrystallized from ethyl acetate/methanol.

In this manner, 0.520g of 2-isopropyl-3-{4-[3-(tert-butylamino)propyloxy]benzenesulphonyl}indolizine hydrochloride was obtained on the form of a white solid.
Yield : 54.3%
M.P. : 198-200 °C.

Using the same procedure as that described above, 2-ethyl-3-{4-[3-(di-n-butylamino)propyloxy]-benzenesulphonyl}indolizine acid oxalate was obtained (SR 33711A) (Example 2).
Yield : 75.5%
M.P. : 71-73 °C (ethyl acetate)

EXAMPLE 7

Preparation of 4-[3-(di-n-butylamino)propyloxy]benzenesulphonylbenzene oxalate (SR 31810A)

a) (4-Hydroxybenzenesulphonyl) benzene

To a solution of O.O5 mol of (4-methoxybenzenesulphonyl)benzene in 150 ml of anhydrous benzene, was added 0.02 mol of aluminium chloride and the reaction medium was maintained for about 15 hours under stirring at room-temperature. After this period of time, the mixture was poured onto crushed ice. The

organic phase was collected, dried on sodium sulphate and evaporated to dryness.

In this manner (4-hydroxybenzenesulphonyl) benzene was obtained in a yield of 68%.

M.P. : 135 °C.

b) 4-[3-(di-n-Butylamino)propyloxy]benzenesulphonylbenzene oxalate

To a solution of 0.0147 mol of (4-hydroxybenzenesulphonyl)benzene in 25ml of dimethylsulphoxide, was added 0.0294 mol of potassium carbonate and 0.0147 mol of di-n-butylamine. The mixture was maintained under stirring for 24 hours at room-temperature and 60ml of water were added. After extraction with ethyl ether, the organic phase was dried and evaporated to dryness to obtain an oily product. The oxalate was formed by adding oxalic acid in ethyl ether to an ethereal solution of the base so provided.

In this manner, 4-[3-(di-n-butylamino)propyloxy]benzenesulphonylbenzene oxalate was obtained in a yield of 48%.

M.P. : 78-81 °C (isopropanol)

EXAMPLE 8

Preparation of 2-n-butyl-1-{4-[3-(di-n-butylamino)propyloxy]benzenesulphonyl}benzimidazole acid oxalate (SR 33631A)

a) 2-n-Butyl-1-(4-bromopropoxy-benzenesulphonyl)benzimidazole

A solution of 0.0035 mol of 4-bromopropoxy-benzimidazole, 0.0035 mol of 2-n-butyl-benzimidazole and 0.0035 mol of triethylamine in 15ml of dioxan was maintained for 7-8 hours under stirring and at room-temperature. The solvent was eliminated under vacuum to obtain a residue which was purified by chromatography on a silica column (eluent : dichloromethane/ethyl acetate 9/1).

In this manner, 2-n-butyl-1-(4-bromopropoxy-benzenesulphonyl)benzimidazole was obtained and was used as such.

Yield : 50%.

b) 2-n-Butyl-1-{4-[3-(di-n-butylamino) propyloxy] benzenesulphonyl } benzimidazole acid oxalate

To a solution of 0.0017 mol of 2-n-butyl-(4-bromopropoxy-benzenesulphonyl) benzimidazole in 15 ml of dimethylsulphoxide was added 0.0034 mol of n-butylamine. The reaction medium was allowed to stand for 17 hours at room-temperature and then poured into 50 ml of water. After extraction, the ethereal phase was dried and evaporated to dryness. The residue so obtained was purified by chromatography on a silica column (eluent : dichloromethane/ethyl acetate) to obtain an oil which was the desired product in the form of the free base (yield : 50%). The oxalate of the base so provided was formed by dissolving the base in question into ethyl ether and adding an ethereal solution of oxalic acid.

In this manner 2-n-butyl-1-{4-[3-(di-n-butylamino) propyloxy]benzenesulphonyl} benzimidazole acid oxalate was obtained after recrystallization from ethanol.

EXAMPLE 9

Preparation of 2-isopropyl-3-{4-[3-(di-n-butylamino) propyloxy]benzenesulphonyl}pyrazolo[1,5-a]pyridine oxalate (SR 33684A)

a) 2-Isopropyl-3-(4-methoxybenzenesulphonyl)pyrazolo[1,5-a]pyridine

A solution of 0.03 mol of 2-isopropyl-pyrazolo[1,5-a]pyridine and 0.03 mol of 4-methoxybenzenesulphonyl chloride in 60 ml of dichloroethane was cooled to -24 °C. After that 0.068 mol of aluminium chloride was added in one fraction and the reaction medium was allowed to return to room-temperature for 3 hours. The mixture was then poured into ice water and distilled in the presence of ethyl acetate. After drying on sodium sulphate, the medium was filtered and concentrated. The solid so obtained was then recrystallized from an ethyl acetate/hexane mixture to provide a product in the form of a white crystalline solid.

In this manner, 0.018 mol of 2-isopropyl-3-(4-methoxybenzenesulphonyl) pyrazolo[1,5-a]pyridine was obtained.

Yield : 60%.
M.P. : 138 - 139 °C.

b) 2-Isopropyl-3-(4-hydroxybenzenesulphonyl)pyrazolo[1,5-a]pyridine

A mixture of 0.012 mol of 2-isopropyl-3-(4-methoxybenzenesulphonyl) pyrazolo[1,5-a]pyridine and 0.054 mol of pyridine hydrochloride was heated at 220 °C for 1 hour. Water was then added and the mixture was distilled in the presence of ethyl acetate. The medium was dried on sodium sulphate, filtered and concentrated. The solid so obtained was then recrystallized from isopropyl ether to provide a white crystalline product.

In this manner, 0.012 mol of 2-isopropyl-3-(4-hydroxybenzenesulphonyl) pyrazolo[1,5-a]pyridine was obtained.
Yield : 99%.
M.P. : 146.2 °C.

c) 2-Isopropyl-3-{4-[3-(di-n-butylamino)propyloxy]benzenesulphonyl}pyrazolo(1,5-a]pyridine oxalate

A mixture of 0.003 mol of 2-isopropyl-3-(4-hydroxybenzenesulphonyl) pyrazolo [1,5-a] pyridine, 0.003 mol of 1-chloro-3-(di-n-butylamino)propane and 0.004 mol of potassium carbonate in 6 ml of N,N-dimethylformamide was stirred for 40 min. at 100 °C. The reaction medium was then poured into water and distilled in the presence of ethyl acetate. After drying on sodium sulphate, the mixture was filtered and concentrated. The residue was then purified on a silica column using an ethyl acetate/hexane 3/7 mixture as eluent. The base so obtained, in oily form, was then treated with an ethereal solution of one equivalent of oxalic acid and the precipitate which formed was filtered out and recrystallized from an ethyl ether/isopropanol mixture.

In this manner, 0.0028 mol of 2-isopropyl-3-{4-[3-(di-n-butylamino) propyloxy]-benzenesulphonyl}pyrazolo[1,5-a]pyridine oxalate was obtained.
Yield : 92%.
M.P. : 72 °C.

EXAMPLE 10

Preparation of 2-isopropyl-3-{4-[3-(tertiobutylamino)propyloxy]benzenesulphonyl}pyrazolo[1,5-a]pyridine oxalate (SR 33686A)

a) 2-Isopropyl-3-[4-(3-bromopropyloxy)benzenesulphonyl]pyrazolo[1,5-a] pyridine

A mixture of 0.003 mol of 2-isopropyl-3-(4-hydroxybenzenesulphonyl) pyrazolo[1,5-a]pyridine, 0.064 mol of 1,3-dibromopropane and 0.004 mol of potassium carbonate in 6 ml of N,N-dimethylformamide was stirred at 100 °C for one hour. The medium was then poured into water and distilled in the presence of ethyl acetate. After drying on sodium sulphate, the mixture was filtered and concentrated to obtain a residue which was purified on a silica column using an ethyl acetate/hexane 1/1 mixture.

In this manner, 0.0021 mol of 2-isopropyl-3-[4-(3-bromopropyloxy) benzenesulphonyl]pyrazolo[1,5-a]-pyridine was obtained in the form of a viscous oil.
Yield : 69%.

b) 2-Isopropyl-3-{4-[3-(tertiobutylamino)propyloxy]benzenesulphonyl}pyrazolo[1,5-a]pyridine oxalate

In a flask, a solution of 0.002 mol of 2-isopropyl-3-[4-(bromopropyloxy)benzenesulphonyl]pyrazolo[1,5-a]pyridine and 0.008 mol of tertiobutylamine in 4 ml of N,N-dimethylsulphoxide was stirred at room-temperature for 24 hours. The mixture was poured into water and then distilled in the presence of ethyl acetate. After drying on sodium sulphate, the medium was filtered and concentrated to obtain a base in oily form. A solution of this base in an ethyl ether/ethyl acetate mixture was then treated with one equivalent of oxalic acid and the white precipitate so obtained was recrystallized from an ethyl ether/isopropanol mixture.

In this manner, 0.002 mol of 2-isopropyl-3-{4-[3-(tertiobutylamino) propyloxy]-benzenesulphonyl}pyrazolo[1,5-a]pyridine oxalate was obtained.
Yield : 99%.
M.P. : 208 °C.

EXAMPLE 11

Preparation of 2-isopropyl-3-{4-[3-(di-n-butylamino)propyloxy]benzenesulphonyl}quinoline oxalate (SR 33695 A)

a) 2-Isopropyl-3-(4-tosyloxybenzenesulphonyl)quinoline

In a sealed tube a mixture of 0.02 mol of 2-aminobenzaldehyde and 0.02 mol of 1-(4-tosyloxybenzenesulphonyl)-3-methyl-butan-2-one was heated at 185 ° C for 2 hours. The mixture was then taken up in dry ethyl ether and filtered.
M.P. of the hydrochloride : about 90 ° C.

b) 2-Isopropyl-3-(4-hydroxybenzenesulphonyl)quinoline

To a solution of 0.017 mol of 2-isopropyl-3-(4-tosyloxybenzenesulphonyl) quinoline in 250 ml of ethanol, was added a solution of 0.068 mol of sodium hydroxide in 5 ml of water. The mixture was heated to reflux for 2 hours and then the solvent was eliminated. The residue so obtained was taken up in water and neutralized with acetic acid. The precipitate which formed was then filtered out, dried and recrystallized from a di-chloroethane/heptane 1/1 mixture.

In this manner, 2-isopropyl-3-(4-hydroxybenzenesulphonyl)quinoline was obtained in a yield of 58%.
M.P. : 185 ° C.

c) 2-Isopropyl-3-{4-[3-(di-n-butylamino)propyloxy]benzenesulphonyl} quinoline oxalate

To a solution of 0.005 mol of 2-isopropyl-3-(4-hydroxybenzenesulphonyl)quinoline in 25ml of dimethylsulphoxide, was added 0.015 mol of anhydrous potassium carbonate. The mixture was stirred for 30 min. and 0.0075 mol of 1-chloro-3-(di-n-butylamino)propane was added. The reaction medium was maintained under stirring for 24 hours. After this period of time, the mixture was poured into water and extracted with ethyl ether.
The organic phase was washed with water, dried on sodium sulphate, filtered and evaporated to dryness. An oily base was so provided which was purified by chromatography on a silica column (eluent : isopropanol) and transformed into an oxalate by adding oxalic acid in ethyl ether.

In this manner, 2-isopropyl-3-{4-[3-(di-n-butylamino)propyloxy] benzenesulphonyl}quinoline oxalate was obtained in a yield of 55%.
M.P. : 130 ° C (ethanol)

Using the same method as that described above, 2-isopropyl-3-{4-[3-(tert-butylamino)propyloxy]-benzenesulphonyl}quinoline acid oxalate (SR 33725A) (Example 12) was obtained.
M.P. : 118-120 ° C (isopropanol)

EXAMPLE 13

Preparation of 2-isopropyl-3-{4-[3-(di-n-butylamino)propyloxy]benzenesulphonyl}furan oxalate (SR 33701A)

a) 2-Isopropyl-3-(4-tosyloxybenzenesulphonyl)-4,5-dihydro-furan

A mixture of 0.008 mol of 1-isobutyryl-1-(4-tosyloxybenzenesulphonyl) cyclopropane and 0.022 mol of tricaprylylmethyl ammonium chloride was heated at 130 ° C for 30 min. The reaction mixture wan then chromatographed on a silica column using an ethyl acetate/hexane 25/75 mixture as eluent.

In this manner, 0.0145 mol of 2-isopropyl-3-(4-tosyloxybenzenesulphonyl)-4,5-dihydro-furan was obtained in the form of a white solid.
Yield : 66 %.
M.P. : 103 ° C (ethyl acetate/hexane).

b) 2-Isopropyl-3-(4-tosyloxybenzenesulphonyl) furan

A mixture of 0.035 mol of 2-isopropyl-3-(4-tosyloxybenzenesulphonyl)-4,5-dihydro-furan, 1 mol of manganese dioxide and 3 Å-molecular screen in powder (previously dried at 140 ° C under 0.01mm Hg for 5 h) in 400ml of dry ethyl ether was stirred for 66 hours at room-temperature. The mixture was then filtered

and the solid was rinsed with dichloromethane. After concentration, the medium was chromatographed on a silica column using an ethyl acetate/hexane 2/8 mixture.

In this manner, 0.009 mol of 2-isopropyl-3-(4-tosyloxybenzenesulphonyl)furan was obtained in a yield of 25%.

M.P. : 94 ° C (ethyl acetate/hexane).

c) 2-Isopropyl-3-(4-hydroxybenzenesulphonyl)furan

To a solution of 0.008 mol of 2-isopropyl-3-(4-tosyloxybenzenesulphonyl)furan in 1.8 ml of ethanol were added 18ml of 1N-sodium hydroxide. The milky solution was stirred under reflux to complete dissolution (2 minutes) and the reaction medium was cooled and neutralized with dilute hydrochloric acid. After that, the mixture was distilled in the presence of ethyl acetate. The organic phase was dried on sodium sulphate, filtered and concentrated. The residue so obtained was then purified on a silica column and eluted using an ethyl acetate/hexane 4/6 mixture.

In this manner, 0.0073 mol of 2-isopropyl-3-(4-hydroxybenzenesulphonyl) furan was obtained in a yield of 91%.

M.P. : 131 ° C (ethyl acetate/hexane).

d) 2-Isopropyl-3-{4-[3-(di-n-butylamino)propyloxy]benzenesulphonyl}furan oxalate

A mixture of 0.003 mol of 2-isopropyl-3-(4-hydroxybenzenesulphonyl) furan, 0.003 mol of di-n-butylamine and 3.23 x $10^{-3}$ mols of crushed potassium carbonate in 3ml of N,N-dimethylformamide was heated at 100 ° C for 30 minutes. The mixture was then poured into water and distilled in the presence of ethyl acetate. After drying on sodium sulphate, the medium was filtered and concentrated. The residue so obtained was then purified by chromatography on a silica column using methanol as eluent. After that, the base so provided was transformed into oxalate by adding oxalic acid in ethyl ether.

In this manner, 0.00288 mol of 2-isopropyl-3-{4-[3-(di-n-butylamino) propyloxy]benzenesulphonyl}furan was obtained.

Yield : 92%

M.P. : 98 ° C (ethanol/ethyl ether)

EXAMPLE 14

Preparation of 2-isopropyl-3-{4-[3-(tert-butylamino)propyloxy]benzenesulphonyl}furan oxalate (SR 33702A)

a) 2-Isopropyl-3-[4-(3-bromopropyloxy)benzenesulphonyl]furan

A mixture of 0.003 mol of 2-isopropyl-3-(4-hydroxybenzenesulphonyl) furan, 0.06 mol of 1,3-dibromopropane and 0.005 mol of crushed potassium carbonate in 8ml of N,N-dimethylformamide was heated at 100 ° C for 1 hour. The mixture was poured into water and distilled in the presence of ethyl acetate. After drying on sodium sulphate, the medium was filtered and concentrated. The residue so obtained was then purified by chromatography on a silica column using an ethyl acetate/hexane 2/8 mixture.

In this manner, 0.00282 mol of 2-isopropyl-3-[4-(3-bromopropyloxy) benzenesulphonyl]furan was obtained in oily form.

Yield : 94%.

b) 2-Isopropyl-3-{4-[3-(tert-butylamino)proplyoxy]benzenesulphonyl} furan oxalate

A mixture of 0.00282 mol of 2-isopropyl-3-[4-(3-bromopropyloxy) benzenesulphonyl]furan and 0.013 mol of tert-butylamine in 7ml of dimethylsulphoxide was stirred at room-temperature for 24 hours. After that, the mixture was poured into water, distilled in the presence of ethyl acetate, dried on sodium sulphate, filtered and concentrated. The residue so obtained was purified on a silica column using a methanol/ethyl acetate 2/8 mixture as eluent.

The oily base so provided was then treated with an ethereal solution of oxalic acid and the precipitate was recrystallized from ethanol.

In this manner, 0.0023 mol of 2-isopropyl-3-{4-[3-(tert-butylamino)propyloxy]benzenesulphonyl}furan oxalate was obtained in a yield of 82%.

M.P. : 143.6 ° C.

EXAMPLE 17

Preparation of 4-{4-[3-(di-n-butylamino)propyloxy]benzenesulphonyl}-5-isopropyl-3-phenyl-isoxazole (SR 33758)

a) 5-Isopropyl-3-phenyl-4-(4-tosyloxy-benzenesulphonyl)-isoxazole

To a solution of 0.23 g (0.01 at.gr) of sodium in 15 ml of methanol, were added, by small fractions, 3.95 g (0.01 mol) of 1-(4-tosyloxy-benzenesulphonyl)-3-methyl-2-butanone while maintaining the temperature at 10 °C. At the same temperature, 1.55 g (0.01 mol) of benzhydroxamic acid chloride in 15 ml of methanol was added drop-by-drop in 20 minutes. The mixture was still stirred for 1 hour at 10 °C end the temperature was then allowed to increase to 20 °C while stirring for 4 hours. The medium was evaporated to dryness and the residue was suction-filtered and washed with water. The crude product so obtained was recrystallised from about 180 ml of ethanol and then purified by elution chromatography on silica using chloroform as eluent.

In this manner, 2.65 g of 5-isopropyl-3-phenyl-4-(4-tosyloxy-benzene sulphonyl)-isoxazole were obtained in a yield of 53.2%.
Purity : 38.4% (high pressure liquid chromatography)
M.P. : 147.5 - 149 °C.

b) 4-(4-Hydroxy-benzenesulphonyl)-5-isopropyl-3-phenyl-isoxazole

A mixture of 2.5 g (0.005 mol) of 5-isopropyl-3-phenyl-4-(4-tosyloxy benzenesulphonyl)-isoxazole and 0.8 g (0.02 mol) of sodium hydroxide in 20 ml of isopropanol and 10 ml of water was heated under reflux for 2.5 hours. After reacting, the mixture was allowed to cool, diluted with 50 ml of water and acidified with concentrated hydrochloric acid. The product so obtained was suction-filtered and washed with water to obtain 1.4 g of the desired compound which was purified by elution chromatography on silica using chloroform as eluent.

In this manner, 1.12 g of 4-(4-hydroxy-benzenesulphonyl)-5-isopropyl-3-phenyl-isoxazole was obtained in a yield of 65.9%.
M.P. : 173 - 174.5 °C.

c) 4-{4-[3-(di-n-butylamino)propyloxy]benzenesulphonyl}-5-isopropyl-3-phenyl-isoxazole

This compound was obtained following the method described in Example 16.
M.P. : 53.5 °C (toluene)

EXAMPLE 19

Preparation of 2-isopropyl-3-{4-[3-(di-n-butylamino)propyloxy]benzenesulphonyl}indole acid oxalate (SR 38743A

a) 2-isopropyl-3-(4-methoxy-benzenesulphonyl) indole

A solution of 1.4 g (5 x $10^{-3}$ mol) of 2-isopropyl-3-(4-methoxyphenylthio) indole (prepared from 2-isopropylindole and 4-methoxythiophenol in the presence of iodine) in 25 ml of dichloromethane was stirred and cooled to about -5 °C. After that 2.6 g (15 x $10^{-3}$ mol) of 3-chloroperbenzoic acid in 25 ml of dichloromethane was added drop-by-drop. The temperature was then allowed to return to room-temperature and the stirring was maintained for 2 hours. The reaction product was washed with a diluted sodium hydroxide solution and then twice with water. The medium was dried on anhydrous sodium sulphate, filtered and the solvent was evaporated off.

In this manner, 1.3 g of 2 isopropyl-3-(4-methoxy-benzenesulphonyl) indole was obtained after recrystallisation from toluene.
M.P. : 178 °C.

Using the same procedure as that described above, 3-isopropyl-2-(4-methoxy-benzenesulphonyl)indole was prepared.
Yield : 90%.
M.P. : 124 °C.

b) 2-Isopropyl-3-(4-hydroxy-benzenesulphonyl)indole

To a solution of 3.3 g (0.01 mol) of 2-isopropyl-3-(4-methoxybenzenesulphonyl)indole in 20 ml of N,N-dimethylformamide was added a solution of 0.024 mol of a 50% - suspension sodium hydride and 0.012 mol of 2-mercapto-ethanol in 10 ml of N,N-dimethylformamide. The medium was heated to 135 °C for 4 hours and cooled. After that a solution of 0.016 mol of sodium hydride and 0.008 mol of 2-mercapto-ethanol was again added and the mixture was again heated at 135 °C for 3 hours. The reaction medium was then taken up in 50 ml of water, acidified, extracted with ethyl ether and purified by chromatography on silica.

In this manner, 3.6 g of 2-isopropyl-3-(4-hydroxy-benzenesulphonyl) indole were obtained in oily form and the product was crystallized from an ethanol/water mixture.
Yield : 82%.
M.P. : 152 °C.

Using the same procedure as that described above, 3-isopropyl-2-(4-hydroxy-benzenesulphonyl)indole was prepared.
Yield : 46.9%.
M.P. : about 72 °C.

c) 2-Isopropyl-3-{4-[3-(di-n-butylamino)propyloxy]benzenesulphonyl}indole acid oxalate

This compound was obtained in accordance with Example 1.
Yield : 79.4%
M.P. : about 85 °C (ethyl acetate/isopropanol 8/2).

EXAMPLE 20

Preparation of 1-methyl-2-isopropyl-3-{4-[3-(di-n-butylamino)propyloxy]benzenesulphonyl}indole acid oxalate (SR 33744A)

a) 1-Methyl-2-isopropyl-3-(4-methoxy-benzesulphonyl)indole

A solution of 6.6 g (0.02 mol) of 2-isopropyl-3-(4-methoxy-benzenesulphonyl)indole in 30 ml of hexamethylphosphotriamide was cooled to about 0 °C and 1 g (0.022 mol) of a 55% - suspension of sodium hydride was added by smell fractions. After the hydrogen evolution was terminated 2.8 g (0.02 mol) of methyl iodide were introduced. The stirring was maintained at room-temperature for 12 hours ad the medium was poured into water ad extracted with ethyl ether. The ethereal phase was washed with water, dried on ahydrous sodium sulphate and filtered. The ether was then evaporated off.

In this manner, 5.4 g of 1-methyl-2-isopropyl-3-(4-methoxy-benzenesulphonyl)indole were obtained after recrystallisation from isopropanol/hexane 1/1.
Yield : 78.7%.
M.P. : 125 °C.

Using the same procedure as that described above, 1-methyl-3-isopropyl-2-(4-methoxy-benzenesulphonyl)indole was prepared.
Yield : 85%.
M.P. : 125 °C (hexane/isopropanol 9/1)

b) 1-Methyl-2-isopropyl-3-(4-hydroxy-benzenesulphonyl)indole

This compound was obtained according to Example 19 b).
Yield : 87%.
M.P. : 202 °C.

Using the same procedure as that described above, 1-methyl-3-isopropyl-2-(4-hydroxy-benzenesulphonyl)indole was prepared.
Yield : 45.9%.
M.P. : 185 °C (dichlorethane/ethyl acetate 9/1).

c) 1-Methyl-2-isopropyl-3-{4-[3-(di-n-butylamino)propyloxy]benzenesulphonyl} indole acid oxalate

This compound was obtained according to Example 19 c).
Yield : 80%
M.P. : about 90 ° C (isopropanol)

EXAMPLE 21

Preparation of 1-methyl-2-isopropyl-3-[4-{3-(tert-butylamino)propyloxy} benzenesulphonyl]indole (SR 33770)

To a solution of 2.8 g (0.0085 mol) of 1-methyl-2-isopropyl-3-(4-hydroxy-benzenesulphonyl)indole in 100 ml of N,N-dimethyl formamide were added 14 g of anhydrous and crushed potassium carbonate. After that 6.8 g (0.037 mol) of 1,3-dibromopropane were added and the medium was heated at 100 ° C for 0.5 hour. The mixture was poured into water, extracted with ethyl ether and the ethereal fraction was washed with water, dried on anhydrous sodium sulphate, filtered and evaporated to obtain 2.7 g of 1-methyl-3-[4-(3-bromo-propoxy)benzenesulphonyl]-2-isopropyl-indole (yield : 69%). This crude bromopropoxy derivative was dissolved in 60 ml of dimethylsulphoxide and 2.2 g (0.03 mol) of tert-butylamine were added. The medium was then stirred at room-temperature for 48 hours, poured into water and extracted with ethyl ether. The ethereal solution was then washed with water, dried on anhydrous sodium sulphate, filtered and evaporated. The crude product so obtained (2.3 g) was then purified by chromatography on a silica column (solvent : methanol).

In this manner, 1.4 g of 1-methyl-2-isopropyl-3-[4-{3-(tert-butylamino)propyloxy}benzenesulphonyl]-indole was obtained after recrystallization from a heptane/isopropanol 1/1 mixture.
Yield : 42.3%.
M.P. : 145 ° C.

EXAMPLE 22

Preparation of 2-isopropyl-1-{4-[3-(di-n-butylamino)propyloxy]benzenesulphonyl}benzene acid oxalate (SR33722A)

a) Sodium 2-isopropyl-benzenesulphonate

To a solution of 36.5g (0.268 mol) of 2-isopropyl-phenol, 40g (0.268 mol) of N,N-dimethylthiocarbamoyl chloride and 3.1g (0.013 mol) of tri-ethylbenzylammonium chloride in 270ml of toluene was added, at 15 ° C, a solution of 27g (0.67 mol) of sodium hydroxide in 130ml of water. The stirring was maintained at this temperature for 2 hours. The organic fraction was then washed with water and the toluene was evaporated off. An oily residue was so obtained which was purified by-distillation under vacuum (138-140 ° C; 0.5 mm Hg) to provide 34g of 2-isopropyl O-phenyl-dimethylthiocarbamate. This product was then submitted to a transposition reaction by heating at 300 ° C for 3 hours. The crude 2-isopropyl S-phenyl-thiocarbamate so obtained was then taken up in 600ml of formic acid. To the solution so obtained and at a temperature of 15 ° C, were then added 225ml of 30%-hydrogen peroxide. The stirring was maintained for about 12 hours. The formic acid was then distilled under reduced pressure, the oily residue was taken up in water and sodium hydroxide was added to pH = 9. The water was eliminated and the residue was recrystallized from 200ml of boiling water. The cristallization was rendered complete by adding sodium chloride and the precipitate so obtained was dried under vacuum at 60 ° C.

In this manner 24.6g of sodium 2-isopropyl-benzenesulphonate were obtained.
Yield : 70.7%

Using the same procedure as that described above sodium 2-ethyl-benzenesulphonate(yield : 100%) was prepared from 2-ethyl O-phenyl-dimethylthiocarbamate (B.P. : 130-132 ° C; 1 mm Hg).

b) 2-Isopropyl-1-(4-methoxybenzenesulphonyl)-benzene

A mixture of 110ml of methanesulphonic acid and 11g of phosphoric anhydride was heated to about 80 ° C to complete dissolution of the anhydride. After cooling to room-temperature, 9.5g (0.0425 mol) of sodium 2-isopropyl-benzenesulphonate and 4.6g (0.0425 mol) of anisole were added. The medium was heated at 80 ° C for 2 hours, cooled to room-temperature and poured onto ice. After extraction with ethyl

ether, the ethereal fraction was washed with water, dried on anhydrous sodium sulphate and filtered. The ether was eliminated to obtain 9.8g of a crude product which was purified by chromatography on a silica column (solvent : dichloroethane).

In this manner, 5.2g of 2-isopropyl-1-(4-methoxy-benzenesulphonyl)-benzene were obtained, after recrystallization from heptane/isopropanol 95/5.
Yield : 42.2%
M.P. : 100 °C.

Using the same procedure as that described above, 2-ethyl-1-(4-methoxy-benzenesulphonyl)-benzene was obtained.
Yield : 66.6%
M.P. : 71 °C.

c) 2-Isopropyl-1-(4-hydroxy-benzenesulphonyl)-benzene

A mixture of 4.2g (0.0145 mol) of 2-isopropyl-1-(4-methoxy-benzenesulphonyl)-benzene and 42g of pyridine hydrochloride was heated at 220 °C for 0.5 hour. After cooling, the medium was taken up in water and extracted with dichloroethane. The dichloroethane solution was then washed with water and dried on anhydrous sodium sulphate and filtered. The solvent was then eliminated under vacuum to obtain a product which was recrystallized from an ethyl acetate/heptane 2/8 mixture.

In this manner 3.2g of 2-isopropyl-1-(4-hydroxy-benzenesulphonyl)-benzenewere obtained in a yield of 80%.
M.P. : 160 °C.

Using the same procedure, 2-ethyl-1-(4-hydroxy-benzenesulphonyl)-benzene was prepared in a yield of 83%
M.P. : 158 °C (heptane/isopropanol 95/5).

d) 2-Isopropyl-1-[4-{3-[N-methyl-N-(3,4-dimethoxy-β-phenethyl)amino] propyloxy}benzenesulphonyl]-benzene acid oxalate.

This compound wan obtained following the same procedure as that described in Example 3b.
Yield : 88.8%
M.P. : 88 °C (ethyl acetate)

Using the same procedure as that described above, the compound hereunder was prepared :
2-Ethyl-1-{4-[3-(di-n-butylamino)propyloxy]benzenesulphonyl}benzene acid oxalate (SR 33740A) (Example 23)
Yield : 72.4%
M.P. : about 80 °C (isopropanol/ethyl ether).

EXAMPLE 24

Preparation of 2-isopropyl-1-[4-{3-(tert-butylamino)propyloxy}benzene-sulphonyl]benzene acid oxalate (SR 33728A)

a) 2-Isopropyl-1-[4-(3-bromo-propyloxy)benzenesulphonyl]benzene.

While stirring 11.6g of anhydrous and crushed potassium carbonate was added to 2g (0.0072 mol) of 2-isopropyl-1-(4-hydroxy-benzenesulphonyl) benzene in 100ml of N,N-dimethylformamide.
After that, 5.8g (0.0288 mol) of 1,3-dibromopropane were added and the whole was heated at 100 °C for 0.5 hour.
The medium was cooled, poured into water and extracted with ethyl ether.
The ethereal layer was washed with water, dried on anhydrous sodium sulphate and filtered. The ether was then eliminated and the residue was purified by chromatography on a silica column using a dichloroethane/hexane 6/4 mixture.

In this manner, 2.4g of 2-isopropyl-1-[4-(3-bromo-propyloxy)benzenesulphonyl]benzene were obtained in oily form.
Yield : 84%
$n_D^{25}$ : 1.558

Using the same procedure as that described above, 2-ethyl-1-[4-(3-bromo-propyloxy)-

benzenesulphonyl]benzene was obtained in a quantitative yield.

b) 2-Isoproyl-1-[4-{3-(tert-butylamino)propyloxy}benzenesulphonyl]benzene acid oxalate.

A mixture of 2.5g (0.00629 mol) of 2-isopropyl-1-[4-(3-bromo-propyloxy) benzenesulphonyl]benzene, 2.26g (0.031 mol) of tert-butylamine and 25ml of dimethylsulphoxide was stirred at room-temperature for 48 hours. The medium was then poured into water and extracted with ethyl ether.
The ethereal fraction was washed with water, dried on anhydrous sodium sulphate and filtered. The ether was eliminated to obtain 2.kg of a base of which the acid oxalate was formed in ether medium.

In this manner, 1.8g of 2-isopropyl-1-[4{3-(tert-butylamino)propyloxy} benzenesulphonyl]benzene acid oxalate was obtained after recrystallization from ethyl acetate/isopropanol 7/3
Yield : 60%
M.P. : 116 °C.

Using the same procedure 2-ethyl-1-[4-{3-(tert-butylamino)propyloxy} benzenesulphonyl]benzene acid oxalate was prepared (SR 33763A) (Example 25)
Yield : 43.1%
M.P. : 203.7 °C (isopropanol)

EXAMPLE 27

According to known pharmaceutical techniques, a capsule containing the following ingredients was prepared :

| Ingredient | mg |
|---|---|
| Compound of the invention | 100.0 |
| Starches | 99.5 |
| Colloidal silica | 0.5 |
| | 200.0 |

## Claims

**Claims for the following Contracting States : AT, BE, CH, DE, FR, GB, IT, LI, LU, NL, SE**

**1.** An aminoalkoxyphenyl derivative of formula :

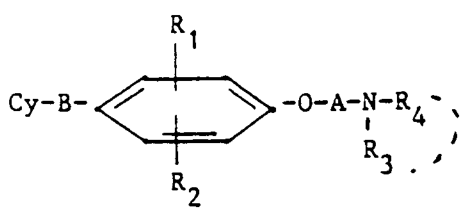

(I)

in which :

| | |
|---|---|
| B | represents a -S-, -SO- or $-SO_2-$ group, |
| $R_1$ and $R_2$ | which are identical or different, each denote hydrogen, a methyl or ethyl radical or a halogen atom, |
| A | denotes a straight- or branched-alkylene radical having from 2 to 5 carbon atoms or a 2-hydroxypropylene radical in which the hydroxy is optionally substituted by a lower alkyl radical, |
| $R_3$ | denotes hydrogen or an alkyl radical in $C_1$-$C_8$, |
| $R_4$ | denotes hydrogen or an alkyl radical in $C_1$-$C_8$; or $R_3$ and $R_4$ when taken together, denote an alkylene or alkenylene radical having from 3 to 6 carbon atoms and optionally substituted with a phenyl radical or optionally interrupted by -O-, |

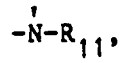

-N= or $R_{11}$ denoting hydrogen or a lower alkyl in $C_1$-$C_4$, phenyl, diphenylmethyl, benzyl or halogenobenzyl radical,

Cy is selected from the group consisting of phenyl optionally substituted with R, in the α position with respect to the methine group directly linked to B;
furyl and isoxazolyl optionally substituted with R in the α position with respect to the methine group directly linked to B and further optionally substituted with one or more groups selected from $C_1$-$C_4$ alkyl and phenyl groups;
benzimidazol-1-yl optionally substituted with R in the second position;
benzimidazol-2-yl optionally substituted with R in the α position with respect to the methine group directly linked to B and further optionally substituted on a nitrogen atom by a radical selected from ($C_1$-$C_4$)alkyl, phenyl, diphenylmethyl, benzyl or halogenobenzyl; and
indolizin-2-yl, indolizin-3-yl, pyrazolo[1,5-a]pyridyl, indolyl and quinolyl optionally substituted with R in the α position with respect to the methine group directly linked to B.

R represents hydrogen, an alkyl radical in $C_1$-$C_8$, or a cycloalkyl radical in $C_3$-$C_6$.

**2.** An aminoalkoxyphenyl derivative according to Claim 1 in which B represents a $-SO_2-$ group.

**3.** An aminoalkoxyphenyl derivative according to claim 1 in which Cy represents an indolizin-3-yl, indolyl, quinolinyl, pyrazolo[1,5-a]pyridyl.

**4.** An aminoalkoxyphenyl derivative according to Claim 1 in which R represents an isopropyl or cyclopropyl group.

**5.** An aminoalkoxyphenyl derivative according to Claim 1 wherein the pharmaceutically acceptable salt is the oxalate or hydrochloride.

**6.** A pharmaceutical or veterinary composition containing, as active principle, at least one aminoalkoxyphenyl derivative according to Claim 1 in combination with a pharmaceutical vehicle or a suitable excipient.

**7.** Process for preparing an aminoalkoxyphenyl derivative according to Claim 1 in which A represents an alkylene radical and B represents a -S- or $-SO_2-$ group, whereby a 4-alkoxyphenyl derivative of general formula :

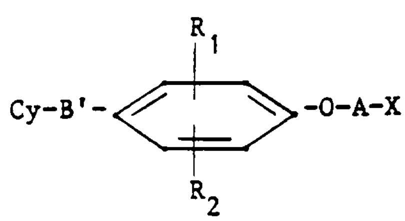

in which B' represents a -S- or $-SO_2-$ group, Cy, B', $R_1$ and $R_2$ have the same meaning as in Claim 1, A represents an alkylene radical as in Claim 1 and X represents a halogen atom, an alkylsulphonyloxy group having from 1 to 4 carbon atoms or an arylsulphonyloxy group having from 6 to 10 carbon atoms is condensed in the presence of an acid acceptor and in a polar or non-polar solvent, with an amine of general formula :

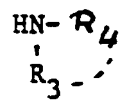

in which $R_3$ and $R_4$ have the same meaning as above, to form the desired aminoalkoxyphenyl derivative in free base form which can, if desired, be reacted with a suitable organic or inorganic acid to form a pharmaceutically acceptable salt thereof.

**8.** Process for preparing a aminoalkoxyphenyl derivative according to Claim 1 in which A represents an alkylene radical and B represents a -S- or $-SO_2-$ group, whereby a 4-hydroxyphenyl derivative of general formula :

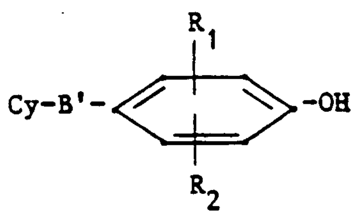

in which B' represents a -S- or $-SO_2-$ group and Cy, $R_1$ and $R_2$ have the same meaning as in Claim 1, is reacted, in the presence of a basic agent, with a compound of general formula :

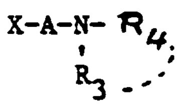

in which X represents a halogen atom, an alkylsulphonyloxy group having from 1 to 4 carbon atoms or an arylsulphonyloxy group having from 6 to 10 carbon atoms, A represents an alkylene radical as in Claim 1 and $R_3$ and $R_4$ have the same meaning as in Claim 1, the reaction taking place at a temperature between room-temperature and the refluxing temperature of the medium and in a polar solvent, to obtain the desired aminoalkoxyphenyl derivative in free base form which can, if desired, be reacted with a suitable organic or inorganic acid to form a pharmaceutically acceptable salt thereof.

**9.** Process for preparing an aminoalkoxyphenyl derivative according to Claim 1 in which B represents a -S- or $-SO_2-$ group and A represents an optionally substituted 2-hydroxy-propylene chain, whereby an oxiranylmethoxy derivative of general formula :

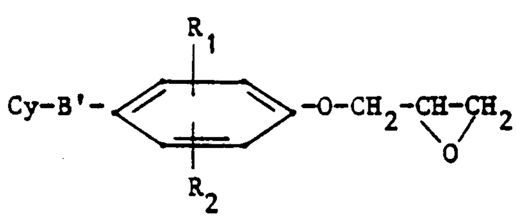

in which Cy, $R_1$ and $R_2$ have the same meaning as in Claim 1 and B' represents a -S- or $-SO_2-$ group is treated under reflux, with an amine of general formula :

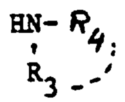

in which $R_3$ and $R_4$ have the same meaning as in Claim 1, this being performed in a polar solvent or in an excess of the said amine :

- to give the desired aminoalkoxyphenyl derivative in the form of the free base in which A represents a 2-hydroxypropylene chain,
- to give an aminoalkoxyphenyl derivative which can be reacted with an alkyl halide having from 1 to 4 carbon atoms in the presence of a strong base to provide the desired aminoalkoxyphenyl derivative in the form of the free base in which A represents a 2-hydroxypropylene chain in which the hydroxy is substituted by an alkyl having from 1 to 4 carbon atoms,

  the aminoalkoxyphenyl derivative so obtained being reacted, if desired, with a suitable organic or inorganic acid to form a pharmaceutically acceptable salt thereof.

**10.** Process for preparing an aminoalkoxyphenyl derivative according to Claim 1 in which B represents a -SO- group, whereby a sulphide of general formula :

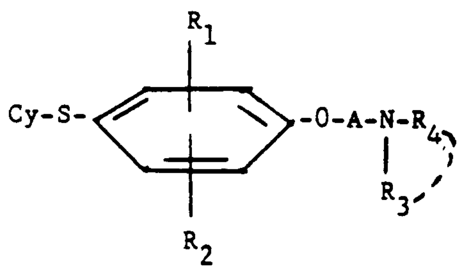

in which Cy, $R_1$, $R_2$, $R_3$, $R_4$ and A have the same meaning as in Claim 1, is treated with an oxidizing agent, this sulphide being in the form of the free base or a salt thereof, to form the desired aminoalkoxyphenyl derivative in free base form which can, if desired, be reacted with a suitable organic or inorganic acid to form a pharmaceutically acceptable salt thereof.

**11.** 2-Isopropyl-3-{4-[3-(tert-butylamino)propyloxy]benzenesulphonyl}indolizine and its N-oxide and pharmaceutically acceptable salts.

**12.** 2-Ethyl-3-{4-[3-(di-n-butylamino)propyloxy]benzenesulphonyl}indolizine and its N-oxide and pharmaceutically acceptable salts.

**13.** 2-Isopropyl-3-{4-[3-(tert-butylamino)propyloxy]benzenesulphonyl}pyrazolo[1, 5-a]pyridine and its N-oxide and pharmaceutically acceptable salts.

**14.** 2-Isopropyl-3-{4-[3-(di-n-butylamino)propyloxy]benzenesulphonyl}quinoline and its N-oxide and pharmaceutically acceptable salts.

**15.** 2-Isopropyl-3-{4-[3-(di-n-butylamino)propyloxy]benzenesulphonyl}furan and its N-oxide and pharmaceutically acceptable salts.

**16.** 2-Isopropyl-3-{4-[3-(di-n-butylamino)propyloxy]benzenesulphonyl}indole and its N-oxide and pharmaceutically acceptable salts.

**17.** 1-Methyl-2-isopropyl-3-{4-[3-(di-n-butylamino)propyloxy]benzenesulphonyl} indole and its N-oxide and pharmaceutically acceptable salts.

**18.** An aminoalkoxyphenyl derivative according to Claim 1 in which $R_1$ and $R_2$ each are hydrogen.

**Claims for the following Contracting State : ES**

1. Process for the preparation of an aminoakoxphenyl derivative of formula :

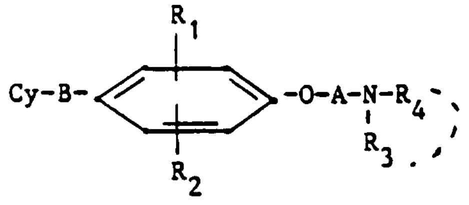

(1)

in which :

| | |
|---|---|
| B | represents a -S-, -SO- or $-SO_2-$ group, |
| $R_1$ and $R_2$ | which are identical or different, each denote hydrogen, a methyl or ethyl radical or a halogen atom, |
| A | denotes a straight- or branched-alkylene radical having from 2 to 5 carbon atoms or a 2-hydroxypropylene radical in which the hydroxy is optionally substituted by a lower alkyl radical, |
| $R_3$ | denotes hydrogen or an alkyl radical in $C_1$-$C_8$, |
| $R_4$ | denotes hydrogen or an alkyl radical in $C_1$-$C_8$; or $R_3$ and $R_4$ when taken together, denote an alkylene or alkenylene radical having from 3 to 6 carbon atoms and optionally substituted with a phenyl radical or optionally interrupted by -O-, -N= or 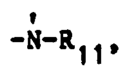 $R_{11}$ denoting hydrogen or a lower alkyl in $C_1$-$C_4$, phenyl, diphenylmethyl, benzyl or halogenobenzyl radical, |
| Cy | is selected from the group consisting of phenyl optionally substituted with R, in the α position with respect to the methine group directly linked to B; furyl and isoxazolyl optionally substituted with R in the α position with respect to the methine group directly linked to B and further optionally substituted with one or more groups selected from $C_1$-$C_4$ alkyl and phenyl groups; benzimidazol-1-yl optionally substituted with R in the second position; benzimidazol-2-yl optionally substituted with R in the α position with respect to the methine group directly linked to B and further optionally substituted on a nitrogen atom by a radical selected from ($C_1$-$C_4$)alkyl, phenyl, diphenylmethyl, benzyl or halogenobenzyl; and indolizin-2-yl, indolizin-3-yl, pyrazolo[1,5-a]pyridyl, indolyl and quinolyl optionally substituted with R in the α position with respect to the methine group directly linked to B. |
| R | represents hydrogen, an alkyl radical in $C_1$-$C_8$, or a cycloalkyl radical in $C_3$-$C_6$, |

said process being characterized in that

a) when A represents an alkylene radical and B represents a -S- or $-SO_2-$ group,

α) a 4-alkoxyphenvl derivative of general formula

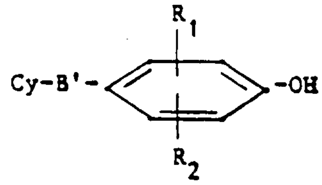

in which B' represents a -S- or $-SO_2-$ group, Cy, ,$R_1$ and $R_2$ have the same meaning as above , A represents an alkylene radical as above and X represents a halogen atom, an alkylsulphonyloxy group having from 1 to 4 carbon atoms or an arylsulphonyloxy group having from 6 to 10 carbon

atoms ,is condensed, in the presence of an acid acceptor and in a polar or non-polar solvent, with an amine of general formula :

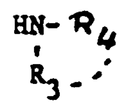

in which $R_3$ and $R_4$ have the same meaning as above, to form the desired aminoalkoxyphenyl derivative in free base form which can, if desired, be reacted with a suitable organic or inorganic acid to form a pharmaceutically acceptable salt thereof.

or $\beta$) a 4-hydroxyphenyl derivative of general formula

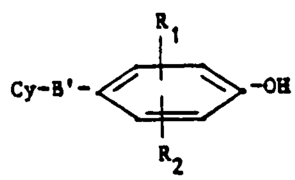

in which B' represents a -S- or $-SO_2-$ group and Cy, $R_1$ and $R_2$ have the same meaning of above, is reacted, in the presence of a basic agent, with a compound of general formula :

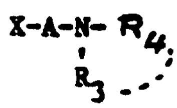

in which X represents a halogen atom, an alkylsulphonyloxy group having from 1 to 4 carbon atoms or a arylsulphonyloxy group having from 6 to 10 carbon atoms, A represents an alkylene radical as above $R_3$ and $R_4$ have the same meaning as above , the reaction taking place at a temperature between room-temperature and the refluxing temperature of the medium and in a polar solvent, to obtain the desired aminoalkoxyphenyl derivative in free base form which can, if desired, be reacted with a suitable organic or inorganic acid to form a pharmaceutically acceptable salt thereof

b) when B represents a -S- or $SO_2$- group ad A represents a optionally substituted 2-hydroxy-propylene chain, an oxiranylmethoxy derivative of general formula :

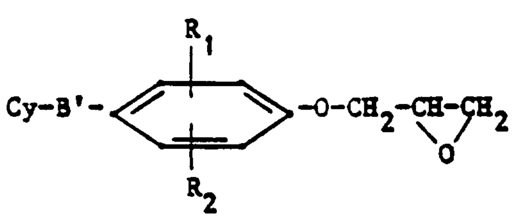

in which Cy, $R_1$ and $R_2$ have the same meaning as in Claim 1 and B' represents a -S- or $-SO_2-$ group is treated under reflux, with an amine of general formula :

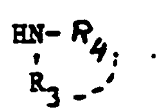

in which $R_3$ and $R_4$ have the same meaning as in Claim 1, this being performed in a polar solvent or in a excess of the said amine :

- to give the desired aminoalkoxyphenyl derivative in the form of the free base in which A represents a 2-hydroxypropylene chain,
- to give an aminoalkoxyphenyl derivative which can be reacted with an alkyl halide having from 1 to 4 carbon atoms ink the presence of a strong base to provide the desired aminoalkoxyphenyl derivative in the form of the free base in which A represents a 2-hydroxypropylene chain in which the hydroxy is substituted by an alkyl having from 1 to 4 carbon atome,

the aminoalkoxyphenyl derivative
so obtained being reacted, if desired, with a suitable organic or inorganic acid to form a pharmaceutically acceptable salt thereof,

c) When B represents a -SO- group, a sulphide of general formula :

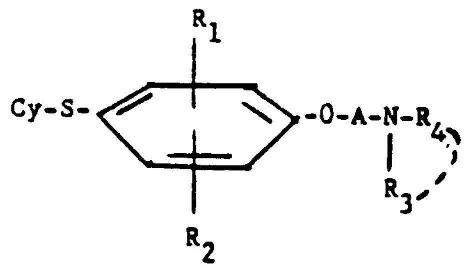

in which Cy, $R_1$, $R_2$, $R_3$, $R_4$ and A have the same meaning as above , is treated with an oxidizing agent, this sulphide being in the form of the free base or a salt thereof, to form the desired aminoalkoxyphenyl derivative in free base form which can, if desired, be reacted with a suitable organic or inorganic acid to form a pharmaceutically acceptable salt thereof.

**2.** A process according to claim 1 in which B represents a $-SO_2-$ group.

**3.** An aminoalkoxyphenyl derivative according to claim 1 in which Cy represents an idolizin-3-yl, indolyl, quinolinyl, pyrazolo[1,5-a]pyridyl.

**4.** A process according to claim 1 in which R represents an isopropyl or cyclopropyl group.

**5.** A process according to claim 1 wherein the pharmaceutically acceptable salt is the oxalate or hydrochloride.

**6.** A process for the preparation of a pharmaceutical composition, comprising putting at least one aminoalkoxyphenyl derivative of the formula (1) defined in claim 1 in a pharmaceutically acceptable form.

**7.** A process for the preparation of a veterinary composition, comprising putting at least one aminoalkoxyphenyl derivative of the formula (1) defined in claim 1 in a pharmaceutically acceptable form.

**Claims for the following Contracting State : GR**

**1.** An aminoalkoxyphenyl derivative of formula :

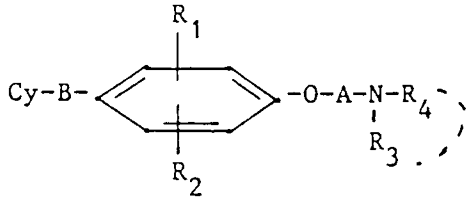

(1)

in which :

B represents a -S-, -SO- or $-SO_2-$ group,

$R_1$ and $R_2$ which are identical or different, each denote hydrogen, a methyl or ethyl radical or a halogen atom,

A denotes a straight- or branched-alkylene radical having from 2 to 5 carbon atoms or a 2-hydroxypropylene radical in which the hydroxy is optionally substituted by a lower alkyl radical,

$R_3$ denotes hydrogen or an alkyl radical in $C_1$-$C_8$,

$R_4$ denotes hydrogen or an alkyl radical in $C_1$-$C_8$; or $R_3$ and $R_4$ when taken together, denote an alkylene or alkenylene radical having from 3 to 6 carbon atoms and optionally substituted with a phenyl radical or optionally interrupted by -O-, -N= or

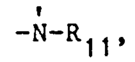

$R_{11}$ denoting hydrogen or a lower alkyl in $C_1$-$C_4$, phenyl, diphenylmethyl, benzyl or halogenobenzyl radical,

Cy is selected from the group consisting of phenyl optionally substituted with R, in the α position with respect to the methine group directly linked to B;
furyl and isoxazolyl optionally substituted with R in the α position with respect to the methine group directly linked to B and further optionally substituted with one or more groups selected from $C_1$-$C_4$ alkyl and phenyl groups;
benzimidasol-1-yl optionally substituted with R in the second position;
benzimidazol-2-yl optionally substituted with R in the α position with respect to the methine group directly linked to B and further optionally substituted on a nitrogen atom by a radical selected from ($C_1$-$C_4$)alkyl, phenyl, diphenylmethyl, benzyl or halogenobenzyl; and
indolizin-2-yl, indolizin-3-yl, pyrazolo[1,5-a]pyridyl, indolyl and quinolyl optionally substituted with R in the α position with respect to the methine group directly linked to B.

R represents hydrogen, an alkyl radical in $C_1$-$C_8$, or a cycloalkyl radical in $C_3$-$C_6$,

2. An aminoalkoxyphenyl derivative according to Claim 1 in which B represents a $-SO_2-$ group.

3. An aminoalkoxyphenyl derivative according to claim 1 in which Cy represents an indolizin-3-yl, indolyl, quinolinyl, pyrazolo[1,5-a]pyridyl.

4. An aminoalkoxyphenyl derivative according to Claim 1 in which R represents an isopropyl or cyclopropyl group.

5. An aminoalkoxyphenyl derivative according to Claim 1 wherein the pharmaceutically acceptable salt is the oxalate or hydrochloride.

6. Process for preparing an aminoalkoxyphenyl derivative according to Claim 1 in which A represents an alkylene radical and B represents a -S- or $-SO_2-$ group, whereby a 4-alkoxyphenyl derivative of general formula :

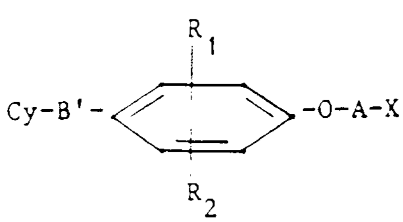

in which B' represents a -S- or $-SO_2-$ group, Cy, $R_1$ and $R_2$ have the same meaning as in Claim 1, A represents an alkylene radical as in Claim 1 and X represents a halogen atom, an alkylsulphonyloxy group having from 1 to 4 carbon atoms or an arylsulphonyloxy group having from 6 to 10 carbon atoms

is condensed in the presence of an acid acceptor and in a polar or non-polar solvent, with an amine of general formula :

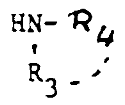

in which $R_3$ and $R_4$ have the same meaning as above, to form the desired aminoalkoxyphenyl derivative in free base form which can, if desired, be reacted with a suitable organic or inorganic acid to form a pharmaceutically acceptable salt thereof.

**7.** Process for preparing a aminoalkoxyphenyl derivative according to Claim 1 in which A represents an alkylene radical and B represents a -S- or $-SO_2-$ group, whereby a 4-hydroxyphenyl derivative of general formula :

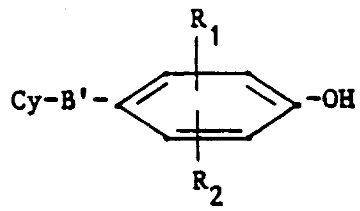

in which B' represents a -S- or $-SO_2-$ group and Cy, $R_1$ and $R_2$ have the same meaning as in Claim 1, is reacted, in the presence of a basic agent, with a compound of general formula :

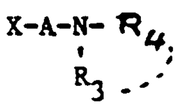

in which X represents a halogen atom, an alkylsulphonyloxy group having from 1 to 4 carbon atoms or an arylsulphonyloxy group having from 6 to 10 carbon atoms, A represents an alkylene radical as in Claim 1 and $R_3$ and $R_4$ have the same meaning as in Claim 1, the reaction taking place at a temperature between room-temperature and the refluxing temperature of the medium and in a polar solvent, to obtain the desired aminoalkoxyphenyl derivative in free base form which can, if desired, be reacted with a suitable organic or inorganic acid to form a pharmaceutically acceptable salt thereof.

**8.** Process for preparing an aminoalkoxphenyl derivative according to Claim 1 in which B represents a -S- or $-SO_2-$ group and A represents an optionally substituted 2-hydroxy-propylene chain, whereby an oxiranylmethoxy derivative of general formula :

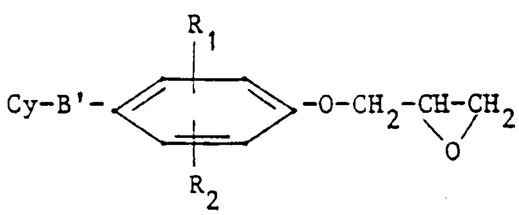

in which Cy, $R_1$ and $R_2$ have the same meaning as in Claim 1 and B' represents a -S- or $-SO_2-$ group is treated under reflux, with an amine of general formula :

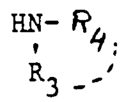

in which $R_3$ and $R_4$ have the same meaning as in Claim 1, this being performed in a polar solvent or in an excess of the said amine :

- to give the desired aminoalkoxyphenyl derivative in the form of the free base in which A represents a 2-hydroxypropylene chain,
- to give an aminoalkoxyphenyl derivative which can be reacted with an alkyl halide having from 1 to 4 carbon atoms in the presence of a strong base to provide the desired aminoalkoxyphenyl derivative in the form of the free base in which A represents a 2-hydroxypropylene chain in which the hydroxy is substituted by an alkyl having from 1 to 4 carbon atoms,

the aminoalkoxyphenyl derivative so obtained being reacted, if desired, with a suitable organic or inorganic acid to form a pharmaceutically acceptable salt thereof.

**9.** Process for preparing an aminoalkoxyphenyl derivative according to Claim 1 in which B represents a -SO- group, whereby a sulphide of general formula :

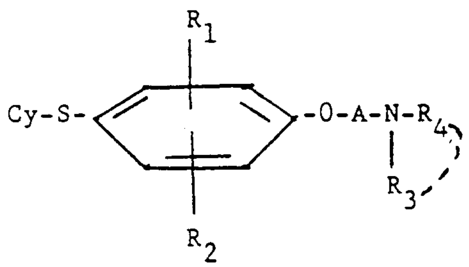

in which Cy, $R_1$, $R_2$, $R_3$, $R_4$ and A have the same meaning as in Claim 1, is treated with an oxidizing agent, this sulphide being in the form of the free base or a salt thereof, to form the desired aminoalkoxyphenyl derivative in free base form which can, if desired, be reacted with a suitable organic or inorganic acid to form a pharmaceutically acceptable salt thereof.

**10.** 2-Isopropyl-3-{4-[3-(tert-butylamino)propyloxy]benzenesulphonyl}indolizine and its N-oxide and pharmaceutically acceptable salts.

**11.** 2-Ethyl-3-{4-[3-(di-n-butylamino)propyloxy]benzenesulphonyl}indolizine and its N-oxide and pharmaceutically acceptable salts.

**12.** 2-Isopropyl-3-{4-[3-(tert-butylamino)propyloxy]benzenesulphonyl}pyrazolo[1, 5-a]pyridine and its N-oxide and pharmaceutically acceptable salts.

**13.** 2-Isopropyl-3-{4-[3-(di-n-butylamino)propyloxy]benzenesulphonyl}quinoline and its N-oxide and pharmaceutically acceptable salts.

**14.** 2-Isopropyl-3-{4-[3-(di-n-butylamino)propyloxy]benzenesulphonyl}furan and its N-oxide and pharmaceutically acceptable salts.

**15.** 2-Isopropyl-3-{4-[3-(di-n-butylamino)propyloxy]benzenesulphonyl}indole and its N-oxide and pharmaceutically acceptable salts.

**16.** 1-Methyl-2-isopropyl-3-{4-[3-(di-n-butylamino)propyloxy]benzenesulphonyl} indole and its N-oxide and pharmaceutically acceptable salts.

**17.** An aminoalkoxyphenyl derivative according to Claim 1 in which $R_1$ and $R_2$ each are hydrogen.

**18.** A process for the preparation of a pharmaceutical composition, comprising putting at least one aminoalkoxyphenyl derivative according to claim 1 in a pharmaceutically acceptable form.

**19.** A process for the preparation of a veterinary composition, comprising putting at least one aminoalkoxyphenyl derivative according to claim 1 in a pharmaceutically acceptable form.

**Patentansprüche**

**Patentansprüche für folgende Vertragsstaaten : AT, BE, CH, DE, FR, GB, IT, LI, LU, NL, SE**

**1.** Aminoalkoxyphenylderivat der Formel:

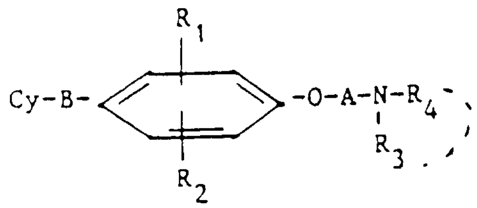

(1)

in der:

| | |
|---|---|
| B | eine Gruppe der Formel -S-,-SO- oder $-SO_2-$, |
| $R_1$ und $R_2$, | die gleichartig oder verschieden sind, jeweils Wasserstoff, eine Methyl- oder Ethylgruppe oder ein Halogenatom, |
| A | eine geradkettige oder verzweigte Alkylengruppe mit 2 bis 5 Kohlenstoffatomen oder eine 2-Hydroxypropylen-gruppe, bei der die Hydroxylgruppe gegebenenfalls durch eine Niedrigalkylgruppe substituiert ist, |
| $R_3$ | Wasserstoff oder eine $C_1$-$C_8$-Alkylgruppe, |
| $R_4$ | Wasserstoff oder eine $C_1$-$C_8$Alkylgruppe oder $R_3$ und $R_4$ gemeinsam eine Alkylen- oder Alkenylen-gruppe mit 3 bis 6 Kohlenstoffatomen, die gegebenenfalls mit einer Phenylgruppe substituiert oder gegebenenfalls durch -O-, -N= oder 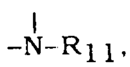 worin $R_{11}$ Wasserstoff oder eine $C_1$-$C_4$-Niedrigalkylgruppe, eine Phenylgruppe, eine Diphenylmethylgruppe, eine Benzylgruppe oder eine Halogenobenzylgruppe darstellt, unterbrochen ist, |
| Cy | eine Gruppe ausgewählt aus der Gruppe, die Phenyl, die gegebenenfalls in der α-Stellung in bezug auf die direkt an B gebundene Methingruppe mit R substituiert ist; Furyl und Isoxazolyl, die gegebenenfalls in der α-Stellung in bezug auf die direkt an B gebundene Methingruppe mit R substituiert sind und gegebenenfalls zusätzlich mit einer oder mehreren Gruppen ausgewählt aus $C_1$-$C_4$-Alkyl- und Phenyl-gruppen substituiert sind; Benzimidazol-1-yl, die gegebenenfalls in der zweiten Stellung mit R substituiert ist; Benzimidazol-2-yl, die gegebenenfalls in der α-Stellung in bezug auf die direkt an B gebundene Methingruppe mit R substituiert ist und gegebenenfalls zusätzlich an einem Stickstoffatom durch eine aus ($C_1$-$C_4$)-Alkyl, Phenyl, Diphenylmethyl, Benzyl oder Halogenobenzyl ausgewählte Gruppe substituiert ist; und Indolizin-2-yl, Indolizin-3-yl, Pyrazolo[1,5-a]pyridyl, Indolyl und Chinolyl, die gegebenenfalls in der α-Stellung in bezug auf die direkt an B gebundene Methingruppe mit R substituiert sind, und |
| R | Wasserstoff, eine $C_1$-$C_8$-Alkylgruppe oder eine $C_3$-$C_6$-Cycloalkylgruppe bedeuten. |

**2.** Aminoalkoxyphenylderivat nach Anspruch 1, worin B eine $-SO_2$-Gruppe darstellt.

**3.** Aminoalkoxyphenylderivat nach Anspruch 1, worin Cy eine Indolizin-3-yl-, Indolyl-, Chinolinyl- oder Pyrazolol[1,5-a]pyridyl-gruppe bedeutet.

4. Aminoalkoxyphenylderivat nach Anspruch 1, worin R eine Isopropyl- oder Cyclopropylgruppe bedeutet.

5. Aminoalkoxyphenylderivat nach Anspruch 1, worin das pharmazeutisch annehmbare Salz das Oxalat oder Hydrochlorid ist.

6. Pharmazeutische oder veterinärmedizinische Zubereitung enthaltend als Wirkstoff mindestens ein Aminoalkoxyphenylderivat nach Anspruch 1 in Kombination mit einem pharmazeutischen Trägermaterial oder einem geeigneten Bindemittel.

7. Verfahren zur Herstellung eines Aminoalkoxyphenylderivats nach Anspruch 1, worin A eine Alkylengruppe und B eine Gruppe der Formel -S- oder $-SO_2-$bedeuten, welches darin besteht, ein 4-Alkoxyphenylderivat der allgemeinen Formel:

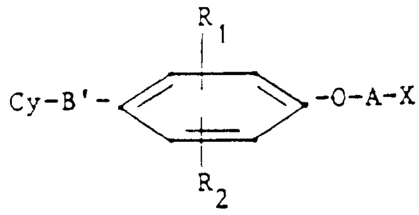

in der B' eine Gruppe der Formel -S- oder $-SO_2-$ darstellt, Cy, $R_1$ und $R_2$ die in Anspruch 1 angegebenen Bedeutungen besitzen, A eine Alkylengruppe darstellt, wie sie in Anspruch 1 definiert worden ist, und X ein Halogenatom, eine Alkylsulfonyloxygruppe mit 1 bis 4 Kohlenstoffatomen oder eine Arylsulfonyloxygruppe mit 6 bis 10 Kohlenstoffatomen darstellt, in Gegenwart eines Säureakzeptors und in einem polaren oder nichtpolaren Lösungsmittel mit einem Amin der allgemeinen Formel:

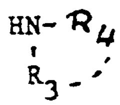

in der $R_3$ und $R_4$ die in Anspruch 1 angegebenen Bedeutungen besitzen, zu kondensieren zur Bildung des gewünschten Aminoalkoxyphenylderivats in Form der freien Base, die gewünschtenfalls mit einer geeigneten organischen oder anorganischen Säure in ein pharmazeutisch annehmbares Salz überfuhrt werden kann.

8. Verfahren zur Herstellung eines Aminoalkoxyphenylderivats nach Anspruch 1, worin A eine Alkylengruppe und B eine Gruppe der Formel -S- oder $-SO_2-$ bedeuten, welches darin besteht, ein 4-Hydroxyphenylderivat der allgemeinen Formel:

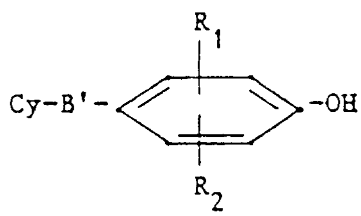

in der B' eine Gruppe der Formel -S- oder $-SO_2-$ darstellt und Cy, $R_1$ und $R_2$ die in Anspruch 1 angegebenen Bedeutungen besitzen, in Gegenwart eines basischen Mittels mit einer Verbindung der allgemeinen Formel:

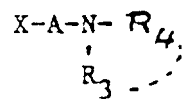

in der X ein Halogenatom, eine Alkylsulfonyloxygruppe mit 1 bis 4 Kohlenstoffatomen oder eine Arylsulfonyloxygruppe mit 6 bis 10 Kohlenstoffatomen bedeutet, A eine Alkylengruppe darstellt, wie sie in Anspruch 1 definiert worden ist, und $R_3$ und $R_4$ die in Anspruch 1 angegebenen Bedeutungen besitzen, umzusetzen, wobei die Reaktion bei einer Temperatur zwischen Raumtemperatur und derRückflußtemperatur des Mediums und in einem polaren Lösungsmittel erfolgt unter Erhalt des gewünschten Aminoalkoxyphenylderivats in Form der freien Base, die gewünschtenfalls mit einer geeigneten organischen oder anorganischen Säure in ein pharmazeutisch annehmbares Salz überführt werden kann.

**9.** Verfahren zur Herstellung eines Aminoalkoxyphenylderivats nach Anspruch 1, worin B eine Gruppe der Formel -S- oder $-SO_2$-und A eine gegebenenfalls substituierte 2-Hydroxypropylenkette bedeuten, welches darin besteht, ein Oxiranylmethoxyderivat der allgemeinen Formel:

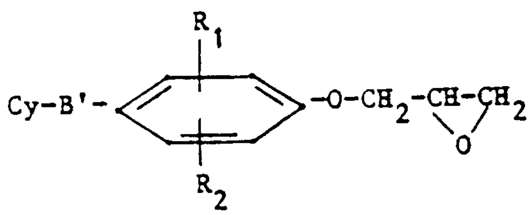

worin Cy, $R_1$ und $R_2$ die in Anspruch 1 angegebenen Bedeutungen besitzen und B' eine Gruppe der Formel -S- oder $-SO_2$- darstellt, unter Rückfluß mit einem Amin der allgemeinen Formel:

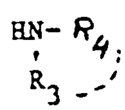

worin $R_3$ und $R_4$ die in Anspruch 1 angegebenen Bedeutungen besitzen, in einem polaren Lösungsmittel oder in einem Überschuß des Amins umzusetzen

- zur Bildung des gewünschten Aminoalkoxyphenylderivats in Form der freien Base, worin A eine 2-Hydroxypropylenkette darstellt, oder
- zur Bildung eines Aminoalkoxyphenylderivats, welches mit einem Alkylhalogenid mit 1 bis 4 Kohlenstoffatomen in Gegenwart einer starken Base umgesetzt werden kann zur Bildung des gewünschten Aminoalkoxyphenylderivats in Form der freien Base, worin A eine 2-Hydroxypropylenkette darstellt, bei der die Hydroxylgruppe durch eine Alkylgruppe mit 1 bis 4 Kohlenstoffatomen substituiert ist.

welches in diese Weise erhaltene Aminoalkoxyphenylderivat gewünschtenfalls mit einer geeigneten organischen oder anorganischen Säure zu einem pharmazeutisch annehmbaren Salz umgewandelt werden kann.

**10.** Verfahren zur Herstellung eines Aminoalkoxyphenylderivats nach Anspruch 1, worin B eine Gruppe der Formel -SO- darstellt, welches darin besteht, ein Sulfid der allgemeinen Formel:

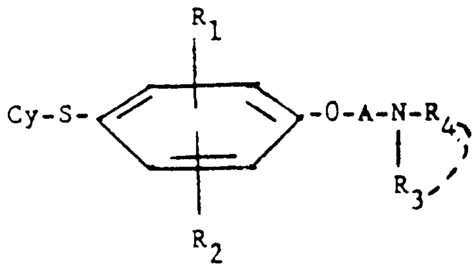

in der Cy, $R_1$, $R_2$, $R_3$, $R_4$ und A die in Anspruch 1 angegebenen Bedeutungen besitzen, in Form der freien Base oder eines Salzes davon mit einem Oxidationsmittel zu behandeln zur Bildung des gewünschten Aminoalkoxyphenylderivats in Form der freien Base, die gegebenenfalls mit einer organischen oder anorganischen Säure in ein pharmazeutisch annehmbares Salz umgesetzt werden kann.

**11.** 2-isopropyl-3-{4-[3-(tetr.-butylamino)-propyloxy]-benzolsulfonyl)-indolizin und dessen N-Oxid und dessen pharmazeutisch annehmbare Salze.

**12.** 2-Ethyl-3-{4[3-(di-n-butylamino)propyloxy]-benzolsulfonyl}-indolizin und dessen N-Oxid und dessen pharmazeutisch annehmbare Salze.

**13.** 2-isopropyl-3-{4-[3-(tetr.-butylamino)-propyloxy]-benzolsulfonyl}-pyrazolo[1,5-a]pyridin und dessen N-Oxid und dessen pharmazeutisch annehmbare Salze.

**14.** 2-Isopropyl-3-{4-[3-(di-n-butylamino)-propyloxy]-benzolsulfonyl}-chinolin und dessen N-Oxid und dessen pharmazeutisch annehmbare Salze.

**15.** 2-Isopropyl-3-{4-[3-(di-n-butylamino)-propyloxy]-benzolsulfonyl}-furan und dessen N-Oxid und dessen pharmazeutisch annehmbare Salze.

**16.** 2-Isopropyl-3-{4-[3-(di-n-butylamino)-propyloxy]-benzolsulfonyl}-indol und dessen N-Oxid und dessen pharmazeutisch annehmbare Salze.

**17.** 1-Methyl-2-isopropyl-3-{4-[3-(di-n-butylamino)-propyloxy]-benzolsulfonyl}-indol und dessen N-Oxid und dessen pharmazeutisch annehmbare Salze.

**18.** Aminoalkoxyphenylderivat nach Anspruch 1, worin $R_1$ und $R_2$ jeweils Wasserstoff bedeuten.

**Patentansprüche für folgenden Vertragsstaat : GR**

**1.** Aminoalkoxyphenylderivat der Formel:

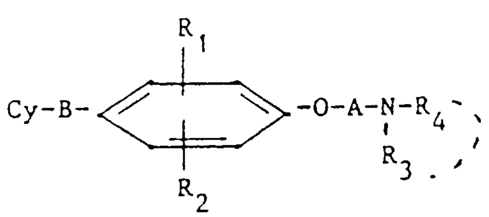

(1)

in der:

| | |
|---|---|
| B | eine Gruppe der Formel -S-, -SO- oder $-SO_2-$, |
| $R_1$ und $R_2$, | die gleichartig oder verschieden sind, jeweils Wasserstoff, eine Methyl- oder Ethylgruppe oder ein Halogenatom, |
| A | eine geradkettige oder verzweigte Alkylengruppe mit 2 bis 5 Kohlenstoffatomen oder eine 2-Hydroxypropylen-gruppe, bei der die Hydroxylgruppe gegebenenfalls durch |

eine Niedrigalkylgruppe substituiert ist,

$R_3$ Wasserstoff oder eine $C_1$-$C_8$-Alkylgruppe,

$R_4$ Wasserstoff oder eine $C_1$-$C_8$Alkylgruppe oder $R_3$ und $R_4$ gemeinsam eine Alkylen- oder Alkenylen-gruppe mit 3 bis 6 Kohlenstoffatomen, die gegebenenfalls mit einer Phenylgruppe substituiert oder gegebenenfalls durch -O-, -N= oder

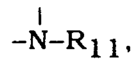

worin $R_{11}$ Wasserstoff oder eine $C_1$-$C_4$-Niedrigalkylgruppe, eine Phenylgruppe, eine Diphenylmethylgruppe, eine Benzylgruppe oder eine Halogenobenzylgruppe darstellt, unterbrochen ist,

Cy eine Gruppe ausgewählt aus der Gruppe, die Phenyl, die gegebenenfalls in der α-Stellung in bezug auf die direkt an B gebundene Methingruppe mit R substituiert ist;
Furyl und Isoxazolyl, die gegebenenfalls in der α-Stellung in bezug auf die direkt an B gebundene Methingruppe mit R substituiert sind und gegebenenfalls zusätzlich mit einer oder mehreren Gruppen ausgewählt aus $C_1$-$C_4$-Alkyl- und Phenyl-gruppen substituiert sind;
Benzimidazol-1-yl, die gegebenenfalls in der zweiten Stellung mit R substituiert ist;
Benzimidazol-2-yl, die gegebenenfalls In der α-Stellung in bezug auf die direkt an B gebundene Methingruppe mit R substituiert ist und gegebenenfalls zusätzlich an einem Stickstoffatom durch eine aus ($C_1$-$C_4$)-Alkyl, Phenyl, Di-phenylmethyl, Benzyl oder Halogenobenzyl ausgewählte Gruppe substituiert ist; und
Indolizin-2-yl, Indolizin-3-yl, Pyrazolo[1,5-a]pyridyl, Indolyl und Chinolyl, die gegebenenfalls in der α-Stellung in bezug auf die direkt an B gebundene Methingruppe mit R substituiert sind, und

R Wasserstoff, eine $C_1$-$C_8$-Alkylgruppe oder eine $C_3$-$C_6$-Cycloalkylgruppe bedeuten.

**2.** Aminoalkoxyphenylderivat nach Anspruch 1, worin B eine -$SO_2$-Gruppe darstellt.

**3.** Aminoalkoxyphenylderivat nach Anspruch 1, worin Cy eine Indolizin-3-yl-, Indolyl-, Chinolinyl- oder Pyrazolo[1,5-a]pyridyl-gruppe bedeutet.

**4.** Aminoalkoxyphenylderivat nach Anspruch 1, worin R eine Isopropyl- oder Cyclopropylgruppe bedeutet.

**5.** Aminoalkoxyphenylderivat nach Anspruch 1, worin das pharmazeutisch annehmbare Salz das Oxalat oder Hydrochlorid ist.

**6.** Verfahren zur Herstellung eines Aminoalkoxyphenylderivats nach Anspruch 1 , worin A eine Alkylengruppe und B eine Gruppe der Formel -S- oder -$SO_2$- bedeuten, welches darin besteht, ein 4-Alkoxyphenylderivat der allgemeinen Formel:

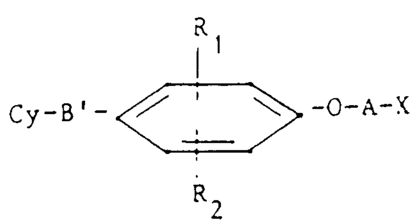

in der B' eine Gruppe der Formel -S- oder -$SO_2$- darstellt, Cy, $R_1$ und $R_2$ die in Anspruch 1 angegebenen Bedeutungen besitzen, A eine Alkylengruppe darstellt, wie sie in Anspruch 1 definiert worden ist, und X ein Halogenatom, eine Alkysulfonyloxygruppe mit 1 bis 4 Kohlenstoffatomen oder eine Arylsulfonyloxygruppe mit 6 bis 10 Kohlenstoffatomen darstellt, in Gegenwart eines Säureakzeptors und in einem polaren oder nichtpolaren Lösungsmittel mit einem Amin der allgemeinen Formel:

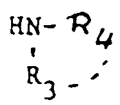

in der $R_3$ und $R_4$ die in Anspruch 1 angegebenen Bedeutungen besitzen, zu kondensieren zur Bildung des gewünschten Aminoalkoxyphenylderivats in Form der freien Base, die gewünschtenfalls mit einer geeigneten organischen oder anorganischen Säure in ein pharmazeutisch annehmbares Salz überführt werden kann.

**7.** Verfahren zur Herstellung eines Aminoalkoxyphenylderivats nach Anspruch 1, worin A eine Alkylengruppe und B eine Gruppe der Formel -S- oder $-SO_2-$ bedeuten, welches darin besteht, ein 4-Hydroxyphenylderivat der allgemeinen Formel:

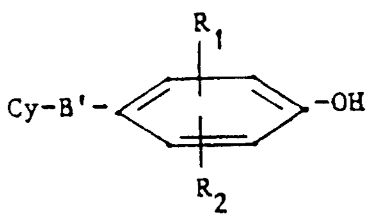

in der B' eine Gruppe der Formel -S- oder $-SO_2-$ darstellt und Cy, $R_1$ und $R_2$ die in Anspruch 1 angegebenen Bedeutungen besitzen, in Gegenwart eines basischen Mittels mit einer Verbindung der allgemeinen Formel:

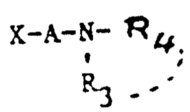

in der X ein Halogenatom, eine Alkylsulfonyloxygruppe mit 1 bis 4 Kohlenstoffatomen oder eine Arylsulfonyloxygruppe mit 6 bis 10 Kohlenstoffatomen bedeutet, A eine Alkylengruppe darstellt, wie es in Anspruch 1 definiert worden ist, und $R_3$ und $R_4$ die in Anspruch 1 angegebenen Bedeutungen besitzen, umzusetzen, wobei die Reaktion bei einer Temperatur zwischen Raumtemperatur und der Rückflußtemperatur des Mediums und in einem polaren Lösungsmittel erfolgt unter Erhalt des gewünschten Aminoalkoxyphenylderivats in Form der freien Base, die gewünschtenfalls mit einer geeigneten organischen oder anorganischen Säure in ein pharmazeutisch annehmbares Salz überführt werden kann.

**8.** Verfahren zur Herstellung eines Aminoalkoxyphenylderivats nach Anspruch 1, worin B eine Gruppe der Formel -S-oder $-SO_2-$ und A eine gegebenenfalls substituierte 2-Hydroxypropylenkette bedeuten, welches darin besteht, ein Oxiranylmethoxyderivat der allgemeinen Formel:

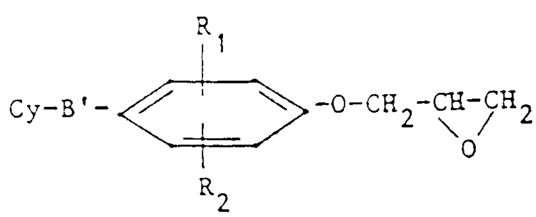

worin Cy, $R_1$ und $R_2$ die in Anspruch 1 angegebenen Bedeutungen besitzen und B' eine Gruppe der Formel -S- oder $-SO_2-$ darstellt, unter Rückfluß mit einem Amin der allgemeinen Formel:

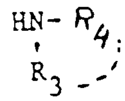

worin $R_3$ und $R_4$ die in Anspruch 1 angegebenen Bedeutungen besitzen, in einem polaren Lösungsmittel oder in einem Überschuß des Amins umzusetzen

- zur Bildung des gewünschten Aminoalkoxyphenylderivats in Form der freien Base, worin A eine 2-Hydroxypropylenkette darstellt, oder
- zur Bildung eines Aminoalkoxyphenylderivats, welches mit einem Alkylhalogenid mit 1 bis 4 Kohlenstoffatomen in Gegenwart einer starken Base umgesetzt werden kann zur Bildung des gewünschten Aminoalkoxyphenylderivats in Form der freien Base, worin A eine 2-Hydroxypropylenkette darstellt, bei der die Hydroxylgruppe durch eine Alkylgruppe mit 1 bis 4 Kohlenstoffatomen substituiert ist,

welches in diese Weise erhaltene Aminoalkoxyphenylderivat gewünschtenfalls mit einer geeigneten organischen oder anorganischen Säure zu einem pharmazeutisch annehmbaren Salz umgewandelt werden kann.

**9.** Verfahren zur Herstellung eines Aminoalkoxyphenylderivats nach Anspruch 1, worin B eine Gruppe der Formel -SO- darstellt, welches darin besteht, ein Sulfid der allgemeinen Formel:

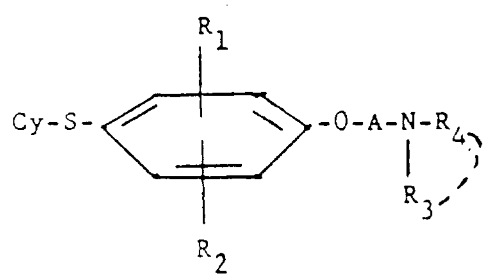

in der Cy, $R_1$, $R_2$, $R_3$, $R_4$ und A die in Anspruch 1 angegebenen Bedeutungen besitzen, in Form der freien Base oder eines Salzes davon mit einem Oxidationsmittel zu behandeln zur Bildung des gewünschten Aminoalkoxyphenylderivats in Form der freien Base, die gegebenenfalls mit einer organischen oder anorganischen Säure in ein pharmazeutisch annehmbares Salz umgesetzt werden kann.

**10.** 2-Isopropyl-3-{4-[3-(tetr.-butylamino)-propyloxy]-benzolsulfonyl}-indolizin und dessen N-Oxid und dessen pharmazeutisch annehmbare Salze.

**11.** 2-Ethyl-3-{4-[3-(di-n-butylamino)-propyloxy]-benzolsulfonyl}-indolizin und dessen N-Oxid und dessen pharmazeutisch annehmbare Salze.

**12.** 2-Isopropyl-3-{4-[3-(tetr.-butylamino)-propyloxy]-benzolsulfonyl}-pyrazolo[1,5-a]pyridin und dessen N-Oxid und dessen pharmazeutisch annehmbare Salze.

**13.** 2-Isopropyl-3-{4-[3-(di-n-butylamino)-propyloxy]-benzolsulfonyl}-chinolin und dessen N-Oxid und dessen pharmazeutisch annehmbare Salze.

**14.** 2-Isopropyl-3-{4-[3-(di-n-butylamino)-propyloxy]-benzolsulfonyl}-furan und dessen N-Oxid und dessen pharmazeutisch annehmbare Salze.

**15.** 2-Isopropyl-3-{4-[3-(di-n-butylamino)-propyloxy]-benzolsulfonyl}-indol und dessen N-Oxid und dessen pharmazeutisch annehmbare Salze.

**16.** 1-Methyl-2-isopropyl-3-{4-[3-(di-n-butylamino)-propyloxyl]-benzolsulfonyl}-indol und dessen N-Oxid und dessen pharmazeutisch annehmbare Salze.

**17.** Aminoalkoxyphenylderivat nach Anspruch 1, worin $R_1$ und $R_2$ jeweils Wasserstoff bedeuten.

**18.** Verfahren zur Herstellung einer pharmazeutischen Zubereitung, welches darin besteht, mindestens ein Aminoalkoxyphenylderivat nach Anspruch 1 in eine pharmazeutisch annehmbare Form zu bringen.

**19.** Verfahren zur Herstellung einer veterinärmedizinischen Zubereitung, welches darin besteht, mindestens ein Aminoalkoxyphenylderivat nach Anspruch 1 in eine pharmazeutisch annehmbare Form zu bringen.

**Patentansprüche für folgenden Vertragsstaat : ES**

**1.** Verfahren zur Herstellung eines Aminoalkoxyphenylderivats der Formel:

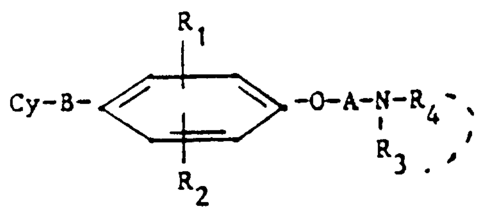

(1)

in der:

| | |
|---|---|
| B | eine Gruppe der Formel -S-, -SO- oder $-SO_2-$, |
| $R_1$ und $R_2$, | die gleichartig oder verschieden sind, jeweils Wasserstoff, eine Methyl- oder Ethylgruppe oder ein Halogenatom, |
| A | eine geradkettige oder verzweigte Alkylengruppe mit 2 bis 5 Kohlenstoffatomen oder eine 2-Hydroxypropylen-gruppe, bei der die Hydroxylgruppe gegebenenfalls durch eine Niedrigalkylgruppe substituiert ist, |
| $R_3$ | Wasserstoff oder eine $C_1$-$C_8$-Alkylgruppe, |
| $R_4$ | Wasserstoff oder eine $C_1$-$C_8$Alkylgruppe oder $R_3$ und $R_4$ gemeinsam eine Alkylen- oder Alkenylen-gruppe mit 3 bis 6 Kohlenstoffatomen, die gegebenenfalls mit einer Phenylgruppe substituiert oder gegebenenfalls durch -O-, -N= oder 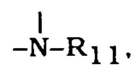 worin $R_{11}$ Wasserstoff oder eine $C_1$-$C_4$-Niedrigalkylgruppe, eine Phenylgruppe, eine Diphenylmethylgruppe, eine Benzylgruppe oder eine Halogenobenzylgruppe darstellt, unterbrochen ist, |
| Cy | eine Gruppe ausgewählt aus der Gruppe, die Phenyl, die gegebenenfalls in der $\alpha$-Stellung in bezug auf die direkt an B gebundene Methingruppe mit R substituiert ist; Furyl und Isoxazolyl, die gegebenenfalls in der $\alpha$-Stellung in bezug auf die direkt an B gebundene Methingruppe mit R substituiert sind und gegebenenfalls zusätzlich mit einer oder mehreren Gruppen ausgewählt aus $C_1$-$C_4$-Alkyl- und Phenyl-gruppen substituiert sind; Benzimidazol-1-yl, die gegebenenfalls in der zweiten Stellung mit R substituiert ist; Benzimidazol-2-yl, die gegebenenfalls in der $\alpha$-Stellung in bezug auf die direkt an B gebundene Methingruppe mit R substituiert ist und gegebenenfalls zusätzlich an einem Stickstoffatom durch eine aus ($C_1$-$C_4$)-Alkyl, Phenyl, Di-phenylmethyl, Benzyl oder Halogenobenzyl ausgewählte Gruppe substituiert ist; und Indolizin-2-yl, Indolizin-3-yl, Pyrazolo[1,5-a]pyridyl, Indolyl und Chinolyl, die gegebenenfalls in der $\alpha$-Stellung in bezug auf die direkt an B gebundene Methingruppe mit R substituiert sind, und |
| R | Wasserstoff, eine $C_1$-$C_8$-Alkylgruppe oder eine $C_3$-$C_6$-Cycloalkylgruppe bedeuten. |

**dadurch gekennzeichnet**, daß man

a) wenn A eine Alkylengruppe und B eine Gruppe der Formel -S- oder $-SO_2-$ bedeuten, man

α) ein 4-Alkoxyphenylderivat der allgemeinen Formel

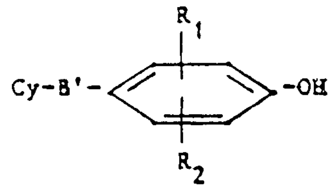

in der B' eine Gruppe der Formel -S- oder $-SO_2-$ darstellt, Cy, $R_1$ und $R_2$ die in Anspruch 1 angegebenen Bedeutungen besitzen, A eine Alkylengruppe darstellt, wie sie in Anspruch 1 definiert worden ist, und X ein Halogenatom, eine Alkylsulfonyloxygruppe mit 1 bis 4 Kohlenstoffatomen oder eine Arylsulfonyloxygruppe mit 6 bis 10 Kohlenstoffatomen darstellt, in Gegenwart eines Säureakzeptors und in einem polaren oder nichtpolaren Lösungsmittel mit einem Amin der allgemeinen Formel:

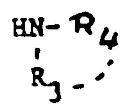

in der $R_3$ und $R_4$ die oben angegebenen Bedeutungen besitzen, kondensiert zur Bildung des gewünschten Aminoalkoxyphenylderivats in Form der freien Base, die gewünschtenfalls mit einer geeigneten organischen oder anorganischen Säure in ein pharmazeutisch annehmbares Salz überführt werden kann; oder

β) ein 4-Hydroxyphenylderivat der allgemeinen Formel

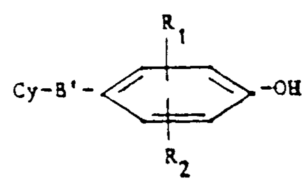

in der B' eine Gruppe der Formel -S- oder $-SO_2-$ darstellt und Cy, $R_1$ und $R_2$ die in Anspruch 1 angegebenen Bedeutungen besitzen, in Gegenwart eines basischen Mittels mit einer Verbindung der allgemeinen Formel:

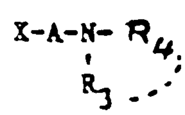

in der X ein Halogenatom, eine Alkylsulfonyloxygruppe mit 1 bis 4 Kohlenstoffatomen oder eine Arylsulfonyloxygruppe mit 6 bis 10 Kohlenstoffatomen bedeutet, A eine Alkylengruppe darstellt, wie sie in Anspruch 1 definiert worden ist, und $R_3$ und $R_4$ die in Anspruch 1 angegebenen Bedeutungen besitzen, umsetzt, wobei die Reaktion bei einer Temperatur zwischen Raumtemperatur und der Rückflußtemperatur des Mediums und in einem polaren Lösungsmittel erfolgt unter Erhalt des gewünschten Aminoalkoxyphenylderivats in Form der freien Base, die gewünschtenfalls mit einer geeigneten organischen oder anorganischen Säure in ein pharmazeutisch annehmbares Salz überführt werden kann; oder

b) wenn B eine Gruppe der Formel -S- oder $-SO_2-$ und A eine gegebenenfalls substituierte 2-Hydroxypropylenkette bedeuten, man ein Oxiranylmethoxyderivat der allgemeinen Formel:

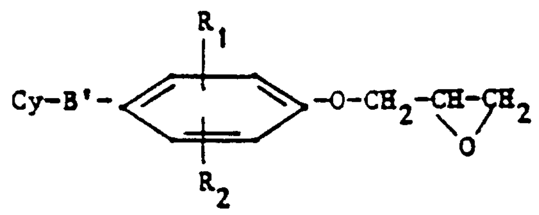

worin Cy, $R_1$ und $R_2$ die in Anspruch 1 angegebenen Bedeutungen besitzen und B' eine Gruppe der Formel -S- oder $-SO_2-$ darstellt, unter Ruckfluß mit einem Amin der allgemeinen Formel:

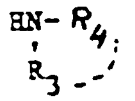

worin $R_3$ und $R_4$ die in Anspruch 1 angegebenen Bedeutungen besitzen, in einem polaren Losungsmittel oder in einem Überschuß des Amins umsetzt

- zur Bildung des gewünschten Aminoalkoxyphenylderivats in Form der freien Base, worin A eine 2-Hydroxypropylenkette darstellt, oder
- zur Bildung eines Aminoalkoxyphenylderivats, welches mit einem Alkylhalogenid mit 1 bis 4 Kohlenstoffatomen in Gegenwart einer starken Base umgesetzt werden kann zur Bildung des gewünschten Aminoalkoxyphenylderivats in Form der freien Base, worin A eine 2-Hydroxypropylenkette darstellt, bei der die Hydroxylgruppe durch eine Alkylgruppe mit 1 bis 4 Kohlenstoffatomen substituiert ist.

welches in diese Weise erhaltene Aminoalkoxyphenylderivat gewünschtenfalls mit einer geeigneten organischen oder anorganischen Säure zu einem pharmazeutisch annehmbaren Salz umgewandelt werden kann; oder

c) wenn B eine Gruppe der Formel -SO- darstellt, man ein Sulfid der allgemeinen Formel:

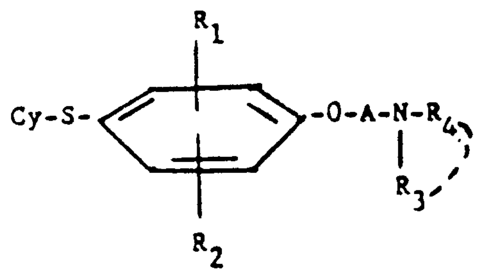

in der Cy, $R_1$, $R_2$, $R_3$, $R_4$ und A die in Anspruch 1 angegebenen Bedeutungen besitzen, in Form der freien Base oder eines Salzes davon mit einem Oxidationsmittel behandelt zur Bildung des gewünschten Aminoalkoxyphenylderivats in Form der freien Base, die gegebenenfalls mit einer organischen oder anorganischen Säure in ein pharmazeutisch annehmbares Salz umgesetzt werden kann.

**2.** Verfahren nach Anspruch 1, worin B eine Gruppe der Formel $-SO_2-$ darstellt.

**3.** Aminoalkoxyphenylderivat nach Anspruch 1 worin Cy eine Indolizin-3-yl-, Indolyl-, Chinolinyl- oder Pyrazolo[1,5-a]pyridyl-gruppe bedeutet.

**4.** Verfahren nach Anspruch 1, worin R eine Isopropyl- oder Cyclopropylgruppe darstellt.

**5.** Verfahren nach Anspruch 1, worin das pharmazeutisch annehmbare Salz das Oxalat oder Hydrochlorid ist.

6. Verfahren zur Herstellung einer pharmazeutischen Zubereitung, welches darin besteht, mindestens ein Aminoalkoxyphenylderivat der Formel (1), wie sie in Anspruch 1 definiert worden ist, in eine pharmazeutisch annehmbare Form bringt.

7. Verfahren zur Herstellung einer veterinärmedizinischen Zubereitung, welches darin besteht, mindestens ein Aminoalkoxyphenylderivat der Formel (1), wie sie in Anspruch 1 definiert worden ist, in eine pharmazeutisch annehmbare Form zu bringen.

**Revendications**

**Revendications pour les Etats contractants suivants : AT, BE, CH, DE, FR, GB, IT, LI, LU, NL, SE**

1. Dérivé aminoalcoxyphényle de formule:

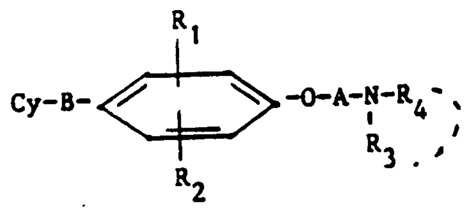

(1)

dans laquelle:

| | |
|---|---|
| B | représente un groupe -S-, -SO- ou $-SO_2-$, |
| $R_1$ et $R_2$, | qui sont identiques ou différents, représentent chacun un atome d'hydrogène, un radical méthyle ou éthyle, ou un atome d'halogène, |
| A | représente un radical alkylène à chaîne droite ou ramifiée possédant de 2 à 5 atomes de carbone ou un radical 2-hydroxypropylène dans lequel le groupe hydroxy est éventuellement substitué par un radical alkyle inférieur, |
| $R_3$ | représente un atome d'hydrogène ou un radical alkyle en $C_1$-$C_8$, |
| $R_4$ | représente un atome d'hydrogène ou un radical alkyle en $C_1$-$C_8$,; ou $R_3$ et $R_4$, pris conjointement, représentent un radical alkylène ou alcénylène comportant de 3 à 6 atomes de carbone et éventuellement substitué par un radical phényle, ou éventuellement interrompu par -O-, -N= ou |

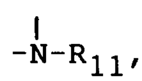

| | |
|---|---|
| | $R_{11}$ représentant un atome d'hydrogène ou un radical alkyle inférieur en $C_1$-$C_4$, phényle, diphénylméthyle, benzyle ou halogénobenzyle, |
| Cy | est choisi dans le groupe constitué par un radical phényle, éventuellement substitué par R, en position $\alpha$ par rapport au groupe méthine fixé directement sur B; un radical furyle et isoxazolyle, éventuellement substitué par R, en position $\alpha$ par rapport au groupe méthine fixé directement sur B et éventuellement substitué aussi par un ou plusieurs groupes choisis parmi les groupes alkyle en $C_1$-$C_4$ et phényle; un radical benzimidazole-1-yle, éventuellement substitué par R en position 2; un radical benzimidazole-2-yle, éventuellement substitué par R, en position $\alpha$ par rapport au groupe méthine fixé directement sur B, et éventuellement substitué aussi sur un atome d'azote par un radical choisi parmi les groupes alkyle en $C_1$-$C_4$, phényle, diphénylméthyle, benzyle ou halogénobenzyle; et les radicaux indolizine-2-yle, indolizine-3-yle, pyrazolo[1,5-a]pyridyle, indolyle et quinoléyle, éventuellement substitués par R, en position $\alpha$ par rapport au groupe méthine fixé directement sur B, |
| R | représente un atome d'hydrogène, un radical alkyle en $C_1$-$C_8$ ou un radical cycloalkyle en $C_3$-$C_6$. |

2. Dérivé aminoalcoxyphényle selon la revendication 1, dans lequel B représente un groupe $-SO_2-$.

3. Dérivé aminoalcoxyphényle selon la revendication 1, dans lequel Cy représente un groupe indolizine-3-yle, indolyle, quinoléinyle, pyrazolo[1,5-a]pyridyle.

4. Dérivé aminoalcoxyphényle selon la revendication 1, dans lequel R représente un groupe isopropyle ou cyclopropyle.

5. Dérivé aminoalcoxyphényle selon la revendication 1, dans lequel le sel pharmaceutiquement acceptable est l'oxalate ou le chlorhydrate.

6. Composition pharmaceutique ou vétérinaire contenant, comme principe actif, au moins un dérivé aminoalcoxyphényle selon la revendication 1, en combinaison avec un véhicule pharmaceutique ou un excipient convenable.

7. Procédé de préparation d'un dérivé aminoalcoxyphényle selon la revendication 1, dans lequel A représente un radical alkylène et B représente un groupe -S- ou $-SO_2-$, qui consiste à condenser un dérivé 4-alcoxyphényle de formule générale:

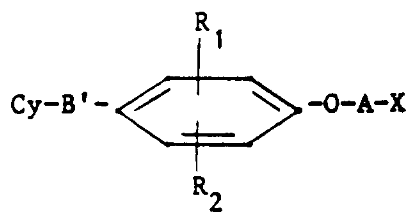

dans laquelle B' représente un groupe -S- ou $-SO_2-$, Cy, $R_1$ et $R_2$ ont la même signification que dans la revendication 1, A représente un radical alkylène tel que dans la revendication 1 et X représente un atome d'halogène, un groupe alkylsulfonyloxy comportant de 1 à 4 atomes de carbone ou un groupe arylsulfonyloxy comportant de 6 à 10 atomes de carbone, en présence d'un accepteur d'acide et dans un solvant polaire ou non polaire, avec une amine de formule générale:

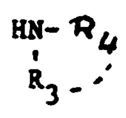

dans laquelle $R_3$ et $R_4$ ont la même signification que ci-dessus, pour former le dérivé aminoalcoxyphényle recherché sous forme de base libre que l'on peut, le cas échéant, faire réagir avec un acide organique ou minéral convenable pour un former un sel pharmaceutiquement acceptable de ce dérivé.

8. Procédé de préparation d'un dérivé aminoalcoxyphényle selon la revendication 1, dans lequel A représente un radical alkylène et B représente un groupe -S- ou $-SO_2-$, qui consiste à faire réagir un dérivé 4-hydroxyphényle de formule générale:

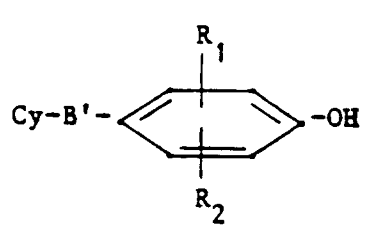

dans laquelle B' représente un groupe -S- ou $SO_2-$, et Cy, $R_1$ et $R_2$ ont la même signification que dans la revendication 1, en présence d'un agent basique, avec un composé de formule générale:

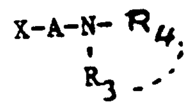

dans laquelle X représente un atome d'halogène, un groupe alkylsulfonyloxy comportant de 1 à 4 atomes de carbone ou un groupe arylsulfonyloxy comportant de 6 à 10 atomes de carbone, A représente un radical alkylène tel que dans la revendication 1 et $R_3$ et $R_4$ ont la même signification que dans la revendication 1, la réaction ayant lieu à une température comprise entre la température ambiante et la température de reflux du milieu et dans un solvant polaire, pour obtenir le dérivé aminoalcoxyphényle recherché sous forme de base libre que l'on peut, le cas échéant, faire réagir avec un acide organique ou minéral convenable pour former un sel pharmaceutiquement acceptable de ce dérivé.

**9.** Procédé de préparation d'un dérivé aminoalcoxyphényle selon la revendication 1, dans lequel B représente un groupe -S- ou $-SO_2-$, et A représente une chaîne 2-hydroxypropylène éventuellement substituée, qui consiste à traiter un dérivé oxiranylméthoxy de formule générale:

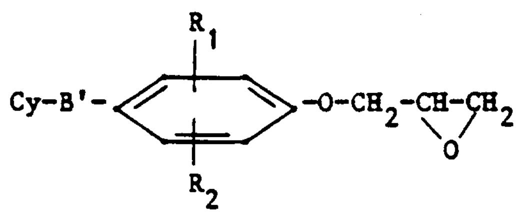

dans laquelle Cy, $R_1$ et $R_2$ ont la même signification que dans la revendication 1, et B' représente un groupe -S- ou $-SO_2-$, au reflux, avec une amine de formule générale:

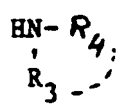

dans laquelle $R_3$ et $R_4$ ont la même signification que dans la revendication 1, la réaction ayant lieu dans un solvant polaire ou dans un excès de ladite amine:

- pour obtenir le dérivé aminoalcoxyphényle recherché sous forme de base libre, dans lequel A représente une chaîne 2-hydroxypropylène,
- pour obtenir un dérivé aminoalcoxyphényle que l'on peut faire réagir avec un halogénure d'alkyle comportant de 1 à 4 atomes de carbone, en présence d'une base forte, pour obtenir le dérivé aminoalcoxyphényle recherché sous forme de base libre, dans lequel A représente une chaîne 2-hydroxypropylène, dans laquelle le groupe hydroxy est substitué par un groupe alkyle comportant de 1 à 4 atomes de carbone, et à faire réagir, le cas échéant, le dérivé aminoalcoxyphényle ainsi obtenu avec un acide organique ou minéral convenable pour un former un sel pharmaceutiquement acceptable de ce dérivé.

**10.** Procédé de préparation d'un dérivé aminoalcoxyphényle selon la revendication 1, dans lequel B représente un groupe -SO-, qui consiste à traiter un sulfure de formule générale:

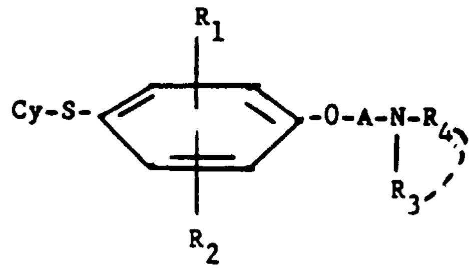

dans laquelle Cy, $R_1$, $R_2$, $R_3$, $R_4$ et A ont la même signification que dans la revendication 1, avec un agent oxydant, ce sulfure étant sous forme de base libre ou d'un de ses sels, pour former le dérivé aminoalcoxyphényle recherché sous forme de base libre que l'on peut, le cas échéant, faire réagir avec un acide organique ou minéral convenable pour former un sel pharmaceutiquement acceptable de ce dérivé.

**11.** 2-Isopropyl-3-{4-[3-(t-butylamino)propyloxy]benzènesulfonyl}indolizine et son N-oxyde et leurs sels pharmaceutiquement acceptables.

**12.** 2-Ethyl-3-{4-[3-(di-n-butylamino)propyloxy]benzènesulfonyl}indolizine et son N-oxyde et leurs sels pharmaceutiquement acceptables.

**13.** 2-Isopropyl-3-{4-[3-(t-butylamino)propyloxy]benzènesulfonyl}pyrazolo[1,5-a]pyridine et son N-oxyde et leurs sels pharmaceutiquement acceptables.

**14.** 2-Isopropyl-3-{4-[3-(di-n-butylamino)propyloxy]benzènesulfonyl}quinoléine et son N-oxyde et leurs sels pharmaceutiquement acceptables.

**15.** 2-Isopropyl-3-{4-[3-(di-n-butylamino)propyloxy]benzènesulfonyl}furane et son N-oxyde et leurs sels pharmaceutiquement acceptables.

**16.** 2-Isopropyl-3-{4-[3-(di-n-butylamino)propyloxy]benzènesulfonyl}indole et son N-oxyde et leurs sels pharmaceutiquement acceptables.

**17.** 1-Méthyl-2-isopropyl-3-{4-[3-(di-n-butylamino)propyloxy]benzènesulfonyl}indole et son N-oxyde et leurs sels pharmaceutiquement acceptables.

**18.** Dérivé aminoalcoxyphényle selon la revendication 1, dans lequel $R_1$ et $R_2$ sont chacun un atome d'hydrogène.

**Revendications pour l'Etat contractant suivant : GR**

**1.** Dérivé aminoalcoxyphényle de formule:

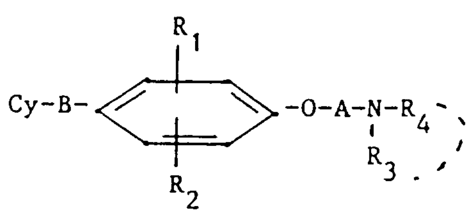

(1)

dans laquelle:

| | |
|---|---|
| B | représente un groupe -S-, -SO- ou $-SO_2-$, |
| $R_1$ et $R_2$, | qui sont identiques ou différents, représentent chacun un atome d'hydrogène, un radical méthyle ou éthyle, ou un atome d'halogène, |
| A | représente un radical alkylène à chaîne droite ou ramifiée possédant de 2 à 5 atomes |

de carbone ou un radical 2-hydroxypropylène dans lequel le groupe hydroxy est éventuellement substitué par un radical alkyle inférieur,

$R_3$ représente un atome d'hydrogène ou un radical alkyle en $C_1$-$C_8$,

$R_4$ représente un atome d'hydrogène ou un radical alkyle en $C_1$-$C_8$,; ou $R_3$ et $R_4$, pris conjointement, représentent un radical alkylène ou alcénylène comportant de 3 à 6 atomes de carbone et éventuellement substitué par un radical phényle, ou éventuellement interrompu par -O-, -N= ou

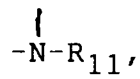

$R_{11}$ représentant un atome d'hydrogène ou un radical alkyle inférieur en $C_1$-$C_4$, phényle, di-phénylméthyle, benzyle ou halogénobenzyle,

Cy est choisi dans le groupe constitué par un radical phényle, éventuellement substitué par R, en position α par rapport au groupe méthine fixé directement sur B;
un radical furyle et isoxazolyle, éventuellement substitué par R, en position α par rapport au groupe méthine fixé directement sur B et éventuellement substitué aussi par un ou plusieurs groupes choisis parmi les groupes alkyle en $C_1$-$C_4$ et phényle;
un radical benzimidazole-1-yle, éventuellement substitué par R en position 2;
un radical benzimidazole-2-yle, éventuellement substitué par R, en position α par rapport au groupe méthine fixé directement sur B, et éventuellement substitué aussi sur un atome d'azote par un radical choisi parmi les groupes alkyle en $C_1$-$C_4$, phényle, diphénylméthyle, benzyle ou halogénobenzyle; et
les radicaux indolizine-2-yle, indolizine-3-yle, pyrazolo[1,5-a]pyridyle, indolyle et quinoléyle, éventuellement substitués par R, en position α par rapport au groupe méthine fixé directement sur B,

R représente un atome d'hydrogène, un radical alkyle en $C_1$-$C_8$ ou un radical cycloalkyle en $C_3$-$C_6$.

**2.** Dérivé aminoalcoxyphényle selon la revendication 1, dans lequel B représente un groupe $-SO_2-$.

**3.** Dérivé aminoalcoxyphényle selon la revendication 1, dans lequel Cy représente un groupe indolizine-3-yle, indolyle, quinoléinyle, pyrazolo[1,5-a]pyridyle.

**4.** Dérivé aminoalcoxyphényle selon la revendication 1, dans lequel R représente un groupe isopropyle ou cyclopropyle.

**5.** Dérivé aminoalcoxyphényle selon la revendication 1, dans lequel le sel pharmaceutiquement acceptable est l'oxalate ou le chlorhydrate.

**6.** Procédé de préparation d'un dérivé aminoalcoxyphényle selon la revendication 1, dans lequel A représente un radical alkylène et B représente un groupe -S- ou $-SO_2-$, qui consiste à condenser un dérivé 4-alcoxyphényle de formule générale:

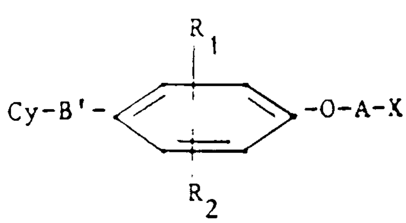

dans laquelle B' représente un groupe -S- ou $-SO_2-$, Cy, $R_1$ et $R_2$ ont la même signification que dans la revendication 1, A représente un radical alkylène tel que dans la revendication 1 et X représente un atome d'halogène, un groupe alkylsulfonyloxy comportant de 1 à 4 atomes de carbone ou un groupe arylsulfonyloxy comportant de 6 à 10 atomes de carbone, en présence d'un accepteur d'acide et dans

un solvant polaire ou non polaire, avec une amine de formule générale:

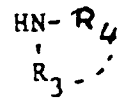

dans laquelle $R_3$ et $R_4$ ont la même signification que ci-dessus, pour former le dérivé aminoalcoxyphényle recherché sous forme de base libre que l'on peut, le cas échéant, faire réagir avec un acide organique ou minéral convenable pour un former un sel pharmaceutiquement acceptable de ce dérivé.

**7.** Procédé de préparation d'un dérivé aminoalcoxyphényle selon la revendication 1, dans lequel A représente un radical alkylène et B représente un groupe -S- ou $-SO_2-$, qui consiste à faire réagir un dérivé 4-hydroxyphényle de formule générale:

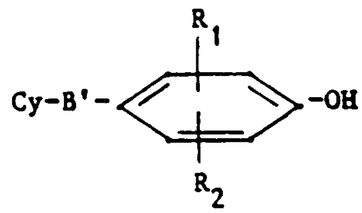

dans laquelle B' représente un groupe -S- ou $-SO_2-$, et Cy, $R_1$ et $R_2$ ont la même signification que dans la revendication 1, en présence d'un agent basique, avec un composé de formule générale:

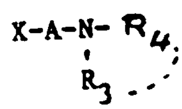

dans laquelle X représente un atome d'halogène, un groupe alkylsulfonyloxy comportant de 1 à 4 atomes de carbone ou un groupe arylsulfonyloxy comportant de 6 à 10 atomes de carbone, A représente un radical alkylène tel que dans la revendication 1 et $R_3$ et $R_4$ ont la même signification que dans la revendication 1, la réaction ayant lieu à une température comprise entre la température ambiante et la température de reflux du milieu et dans un solvant polaire, pour obtenir le dérivé aminoalcoxyphényle recherché sous forme de base libre que l'on peut, le cas échéant, faire réagir avec un acide organique ou minéral convenable pour un former un sel pharmaceutiquement acceptable de ce dérivé.

**8.** Procédé de préparation d'un dérivé aminoalcoxyphényle selon la revendication 1, dans lequel B représente un groupe -S- ou $-SO_2-$, et A représente une chaîne 2-hydroxypropylène éventuellement substituée, qui consiste à traiter un dérivé oxiranylméthoxy de formule générale:

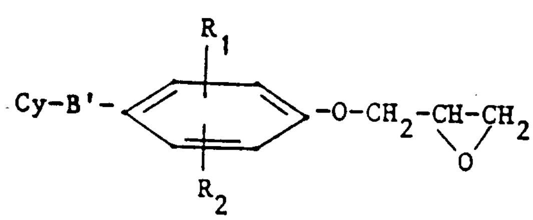

dans laquelle Cy, $R_1$ et $R_2$ ont la même signification que dans la revendication 1, et B' représente un groupe -S- ou $-SO_2-$, au reflux, avec une amine de formule générale:

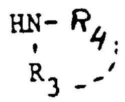

dans laquelle $R_3$ et $R_4$ ont la même signification que dans la revendication 1, la réaction ayant lieu dans un solvant polaire ou dans un excès de ladite amine:

- pour obtenir le dérivé aminoalcoxyphényle recherché sous forme de base libre, dans lequel A représente une chaîne 2-hydroxypropylène,
- pour obtenir un dérivé aminoalcoxyphényle que l'on peut faire réagir avec un halogénure d'alkyle comportant de 1 à 4 atomes de carbone, en présence d'une base forte, pour obtenir le dérivé aminoalcoxyphényle recherché sous forme de base libre, dans lequel A représente une chaîne 2-hydroxypropylène, dans laquelle le groupe hydroxy est substitué par un groupe alkyle comportant de 1 à 4 atomes de carbone, et à faire réagir, le cas échéant, le dérivé aminoalcoxyphényle ainsi obtenu avec un acide organique ou minéral convenable pour un former un sel pharmaceutiquement acceptable de ce dérivé.

**9.** Procédé de préparation d'un dérivé aminoalcoxyphényle selon la revendication 1, dans lequel B représente un groupe -SO-, qui consiste à traiter un sulfure de formule générale:

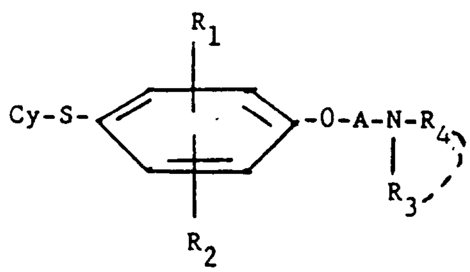

dans laquelle Cy, $R_1$, $R_2$, $R_3$, $R_4$ et A ont la même signification que dans la revendication 1, avec un agent oxydant, ce sulfure étant sous forme de base libre ou d'un de ses sels, pour former le dérivé aminoalcoxyphényle recherché sous forme de base libre que l'on peut, le cas échéant, faire réagir avec un acide organique ou minéral convenable pour un former un sel pharmaceutiquement acceptable de ce dérivé.

**10.** 2-Isopropyl-3-{4-[3-(t-butylamino)propyloxy]benzènesulfonyl}indolizine et son N-oxyde et leurs sels pharmaceutiquement acceptables.

**11.** 2-Ethyl-3-{4-[3-(di-n-butylamino)propyloxy]benzènesulfonyl}indolizine et son N-oxyde et leurs sels pharmaceutiquement acceptables.

**12.** 2-Isopropyl-3-{4-[3-(t-butylamino)propyloxy]benzènesulfonyl}pyrazolo[1,5-a]pyridine et son N-oxyde et leurs sels pharmaceutiquement acceptables.

**13.** 2-Isopropyl-3-{4-[3-(di-n-butylamino)propyloxy]benzènesulfonyl}quinoléine et son N-oxyde et leurs sels pharmaceutiquement acceptables.

**14.** 2-Isopropyl-3-{4-[3-(di-n-butylamino)propyloxy]benzènesulfonyl}furane et son N-oxyde et leurs sels pharmaceutiquement acceptables.

**15.** 2-Isopropyl-3-{4-[3-(di-n-butylamino)propyloxy]benzènesulfonyl}indole et son N-oxyde et leurs sels pharmaceutiquement acceptables.

**16.** 1-Méthyl-2-isopropyl-3-{4-[3-(di-n-butylamino)propyloxy]benzènesulfonyl}indole et son N-oxyde et leurs sels pharmaceutiquement acceptables.

**17.** Dérivé aminoalcoxyphényle selon la revendication 1, dans lequel $R_1$ et $R_2$ sont chacun un atome d'hydrogène.

**18.** Procédé de préparation d'une composition pharmaceutique comprenant le fait de mettre au moins un dérivé aminoalcoxyphényle selon la revendication 1 sous une forme pharmaceutiquement acceptable.

**19.** Procédé de préparation d'une composition vétérinaire comprenant le fait de mettre au moins un dérivé aminoalcoxyphényle selon la revendication 1 sous une forme pharmaceutiquement acceptable.

**Revendications pour l'Etat contractant suivant : ES**

**1.** Procédé de préparation d'un dérivé aminoalcoxyphényle de formule:

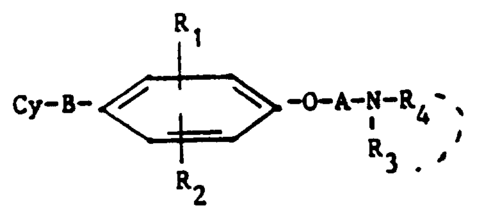

(1)

dans laquelle:

| | |
|---|---|
| B | représente un groupe -S-, -SO- ou $-SO_2-$, |
| $R_1$ et $R_2$, | qui sont identiques ou différents, représentent chacun un atome d'hydrogène, un radical méthyle ou éthyle, ou un atome d'halogène, |
| A | représente un radical alkylène à chaîne droite ou ramifiée possédant de 2 à 5 atomes de carbone ou un radical 2-hydroxypropylène dans lequel le groupe hydroxy est éventuellement substitué par un radical alkyle inférieur, |
| $R_3$ | représente un atome d'hydrogène ou un radical alkyle en $C_1$-$C_8$, |
| $R_4$ | représente un atome d'hydrogène ou un radical alkyle en $C_1$-$C_8$,; ou $R_3$ et $R_4$, pris conjointement, représentent un radical alkylène ou alcénylène comportant de 3 à 6 atomes de carbone et éventuellement substitué par un radical phényle, ou éventuellement interrompu par -O-, -N = ou 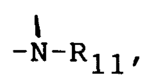 $R_{11}$ représentant un atome d'hydrogène ou un radical alkyle inférieur en $C_1$-$C_4$, phényle, di-phénylméthyle, benzyle ou halogénobenzyle, |
| Cy | est choisi dans le groupe constitué par un radical phényle, éventuellement substitué par R, en position $\alpha$ par rapport au groupe méthine fixé directement sur B; un radical furyle et isoxazolyle, éventuellement substitué par R, en position $\alpha$ par rapport au groupe méthine fixé directement sur B et éventuellement substitué aussi par un ou plusieurs groupes choisis parmi les groupes alkyle en $C_1$-$C_4$ et phényle; un radical benzimidazole-1-yle, éventuellement substitué par R en position 2; un radical benzimidazole-2-yle, éventuellement substitué par R, en position $\alpha$ par rapport au groupe méthine fixé directement sur B, et éventuellement substitué aussi sur un atome d'azote par un radical choisi parmi les groupes alkyle en $C_1$-$C_4$, phényle, diphénylméthyle, benzyle ou halogénobenzyle; et les radicaux indolizine-2-yle, indolizine-3-yle, pyrazolo[1,5-a]pyridyle, indolyle et quinoléyle, éventuellement substitués par R, en position $\alpha$ par rapport au groupe méthine fixé directement sur B, |
| R | représente un atome d'hydrogène, un radical alkyle en $C_1$-$C_8$ ou un radical cycloalkyle en $C_3$-$C_6$, |

ledit procédé étant caractérisé par le fait que

a) lorsque A représente un radical alkylène et a représente un groupe -S- ou $-SO_2-$,

α) il consiste à condenser un dérivé 4-alcoxyphényle de formule générale:

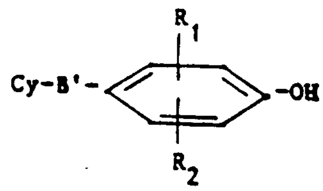

dans laquelle B' représente un groupe -S- ou $-SO_2-$, Cy, $R_1$ et $R_2$ ont la même signification que ci-dessus, A représente un radical alkylène tel que ci-dessus et X représente un atome d'halogène, un groupe alkylsulfonyloxy comportant de 1 à 4 atomes de carbone ou un groupe arylsulfonyloxy comportant de 6 à 10 atomes de carbone, en présence d'un accepteur d'acide et dans un solvant polaire ou non polaire, avec une amine de formule générale:

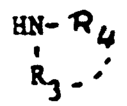

dans laquelle $R_3$ et $R_4$ ont la même signification que ci-dessus, pour former le dérivé aminoalcoxyphényle recherché sous forme de base libre que l'on peut, le cas échéant, faire réagir avec un acide organique ou minéral convenable pour un former un sel pharmaceutiquement acceptable de ce dérivé;

ou β) il consiste à faire réagir un dérivé 4-hydroxyphényle de formule générale:

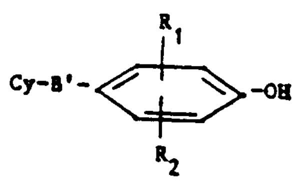

dans laquelle B' représente un groupe -S- ou $-SO_2-$, et Cy, $R_1$ et $R_2$ ont la même signification que ci-dessus, en présence d'un agent basique, avec un composé de formule générale:

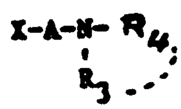

dans laquelle X représente un atome d'halogène, un groupe alkylsulfonyloxy comportant de 1 à 4 atomes de carbone ou un groupe arylsulfonyloxy comportant de 6 à 10 atomes de carbone, A représente un radical alkylène tel que ci-dessus et $R_3$ et $R_4$ ont la même signification que ci-dessus, la réaction ayant lieu à une température comprise entre la température ambiante et la température de reflux du milieu et dans un solvant polaire, pour obtenir le dérivé aminoalcoxyphényle recherché sous forme de base libre que l'on peut, le cas échéant, faire réagir avec un acide organique ou minéral convenable pour un former un sel pharmaceutiquement acceptable de ce dérivé;

b) lorsque B représente un groupe -S- ou $-SO_2-$, et A représente une chaîne 2-hydroxypropylène éventuellement substituée, il consiste à traiter un dérivé oxiranylméthoxy de formule générale:

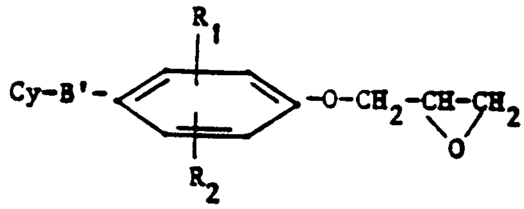

dans laquelle Cy, $R_1$ et $R_2$ ont la même signification que dans la revendication 1, et B' représente un groupe -S- ou $-SO_2-$, au reflux, avec une amine de formule générale:

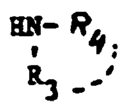

dans laquelle $R_3$ et $R_4$ ont la même signification que dans la revendication 1, la réaction ayant lieu dans un solvant polaire ou dans un excès de ladite amine:

- pour obtenir le dérivé aminoalcoxyphényle recherché sous forme de base libre, dans lequel A représente une chaîne 2-hydroxypropylène,
- pour obtenir un dérivé aminoalcoxyphényle que l'on peut faire réagir avec un halogénure d'alkyle comportant de 1 à 4 atomes de carbone, en présence d'une base forte, pour obtenir le dérivé aminoalcoxyphényle recherché sous forme de base libre, dans lequel A représente une chaîne 2-hydroxypropylène, dans laquelle le groupe hydroxy est substitué par un groupe alkyle comportant de 1 à 4 atomes de carbone, et à faire réagir, le cas échéant, le dérivé aminoalcoxyphényle ainsi obtenu avec un acide organique ou minéral convenable pour un former un sel pharmaceutiquement acceptable de ce dérivé;

c) lorsque B représente un groupe -SO-, il consiste à traiter un sulfure de formule générale:

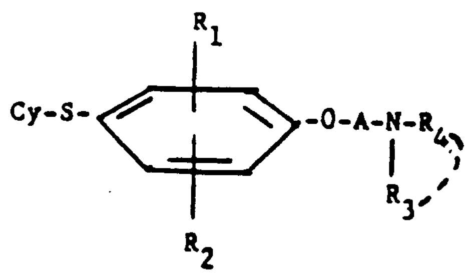

dans laquelle Cy, $R_1$, $R_2$, $R_3$, $R_4$ et A ont la même signification que ci-dessus, avec un agent oxydant, ce sulfure étant sous forme de base libre ou d'un de ses sels, pour former le dérivé aminoalcoxyphényle recherché sous forme de base libre que l'on peut, le cas échéant, faire réagir avec un acide organique ou minéral convenable pour former un sel pharmaceutiquement acceptable de ce dérivé.

**2.** Procédé selon la revendication 1, dans lequel B représente un groupe $-SO_2-$.

**3.** Procédé selon la revendication 1, dans lequel Cy représente un groupe indolizine-3-yle, indolyle, quinoléinyle, pyrazolo[1,5-a]pyridyle.

**4.** Procédé selon la revendication 1, dans lequel R représente un groupe isopropyle ou cyclopropyle.

**5.** Procédé selon la revendication 1, dans lequel le sel pharmaceutiquement acceptable est l'oxalate ou le chlorhydrate.

**6.** Procédé de préparation d'une composition pharmaceutique comprenant le fait de mettre au moins un dérivé aminoalcoxyphényle de formule (I) définie dans la revendication 1 sous une forme pharmaceutiquement acceptable.

**7.** Procédé de préparation d'une composition vétérinaire comprenant le fait de mettre au moins un dérivé aminoalcoxyphényle de formule (I) définie dans la revendication 1 sous une forme pharmaceutiquement acceptable.